# EUROPEAN PATENT APPLICATION

(11) **EP 3 431 493 A1**
(43) Date of publication of application: **23.01.2019**
(21) Application number: 18185624.6
(22) Date of filing: 03.09.2014
(51) Int. Cl.: C07K 14/47, C12N 15/113, A61K 31/713, A61K 38/17, A61P 35/00

(54) **COMPOSITIONS AND METHODS FOR INDUCING SENESCENCE IN CANCER**

(30) Priority: 03.09.2013 EP 13306200
(62) Divisional of application: 14758567.3
(71) Applicant: INSERM (Institut National de la Santé et de la Recherche Médicale), 75013 Paris (FR); Université Nice Sophia Antipolis, 06103 Nice Cedex 2 (FR); Centre Hospitalier Universitaire de Nice, 06000 Nice (FR)
(72) Inventor: PASSERON, Thierry, 06204 Nice Cedex 3 (FR); LE PAPE, Elodie, 06204 Nice Cedex 3 (FR); DE DONATIS, Gian Marco, 06204 Nice Cedex 3 (FR)
(74) Representative: Lavoix

(57) **Abstract**

The invention relates to nucleic acid sequences as well as to modified oligonucleotides (ODNs) comprising a p52 binding site derived from the EZH2 promoter region, as well as to compositions and methods for treating cancer. The invention relates to peptides derived from p52 as well as to compositions and methods for treating cancer. The invention also provides compositions and methods for inducing senescence in cancer cells (e.g. in epithelial cancer cells). The invention also provides compositions and methods for inhibiting expression of EZH2 in cancer cells. The invention further provides a new combination therapy as well as compositions and thereof for treating cancer.

## Description

### FIELD OF THE INVENTION:

The invention relates to the field of oncology. The invention relates to nucleic acid sequences as well as to modified oligonucleotides (ODNs) comprising a p52 binding site derived from the EZH2 promoter region, as well as to compositions and methods for treating cancer. The invention relates to peptides derived from p52 as well as to compositions and methods for treating cancer. The invention also provides compositions and methods for inducing senescence in cancer cells (e.g. in epithelial cancer cells). The invention also provides compositions and methods for inhibiting expression of EZH2 in cancer cells. The invention further provides a new combination therapy as well as compositions and methods for treating cancer.

### BACKGROUND OF THE INVENTION:

Senescence program induces a blockade of cellular proliferation, characteristic morphological changes, and expression of the senescence marker, β-galactosidase (SA-β-gal). Cellular senescence is recognized as a potent tumor suppressor mechanism. Understanding mechanisms that promote senescence could lead to new clinical approaches in the treatment of cancer. Hence, driving cancer cells to undergo senescence represents an opportunity for cancer therapeutics. Furthermore, there are increasing evidences that cellular senescence program can also trigger or potentiate the tumor immune surveillance (Kang et al., 2011; Xue et al., 2007). Using treatments that could restore senescence thus appears very attractive, alone or in combination with immune therapies. Indeed, among current therapeutic strategies, enhancing the immune response against melanoma tumors with ipilimumab, an anti-CTLA4 antibody, has increased the overall survival of stage 4 melanoma patients (Hodi et al., 2010). Interestingly, long term responses can be achieved with such an approach but only 10% of the patients respond to treatment. Thus, the study of the pathways involved in the suppression of senescence program in cancer cells is mandatory to identify targets that could have major therapeutic implications. Moreover, EZH2 is a key oncogenic protein since EZH2 enhances cell cycle progression and suppresses tumor differentiation and senescence program (Chang et al., 2012; Bracken et al., 2007; Kheradmand Kia et al., 2009; Fan et al., 2011). Numerous authors have pointed out the tremendous interest in targeting this protein to limit cancers. The molecular regulation of EZH2 transcription is still poorly understood. Therefore, understanding the molecular mechanisms that control EZH2 expression may have major therapeutical implications.

### SUMMARY OF THE INVENTION:

In a first aspect, the invention relates to an isolated nucleic acid sequence comprising a p52 binding site derived from the EZH2 promoter region, wherein said sequence comprises or consists of the nucleic acid sequence SEQ ID NO: 2, 3, 4 and 5 or any derivative thereof.

In a second aspect, the invention relates to a p52 decoy in the form of an oligonucleotide (ODN) comprising a nucleic acid sequence of a p52 binding site derived from the EZH2 promoter region, wherein said sequence comprises or consists of any one of the nucleic acid sequence of the invention described above.

In a third aspect, the invention relates to a pharmaceutical composition comprising a p52 decoy of the invention and a pharmaceutically acceptable excipient.

In a fourth aspect, the invention relates to an isolated peptide derived from the p52 polypeptide comprising or consisting of the amino acid sequence SEQ ID NO: 10 or a function-conservative variant thereof.

In a sixth aspect, the invention relates to a pharmaceutical composition comprising a peptide of the invention and a pharmaceutically acceptable excipient.

In a seventh aspect, the invention relates to an inhibitor of the non-canonical NF-κB pathway for use in a method for reducing or inhibiting expression of EZH2 in cancer cells.

In an eight aspect, the invention relates to an inhibitor of the non-canonical NF-κB pathway for use in a method for inducing senescence in cancer cells.

In a ninth aspect, the invention relates to inhibitor of non-canonical NF-κB pathway for use in a method for increasing tumor immunogenicity.

In a tenth aspect, the invention relates inhibitor of the non-canonical NF-κB pathway for use in a method for increasing efficacy of a therapy with an immunomodulatory compound.

In another aspect, the invention relates to a pharmaceutical composition or a kit-of-part composition comprising an inhibitor of the non-canonical NF-κB pathway and an immunomodulatory compound.

### DETAILED DESCRIPTION OF THE INVENTION:

The invention relies on the fact that the inventors have now shown that NFκB2/p52 is the master transcription factor of Enhancer of zeste homolog 2 (EZH2), a histone methyltransferase, that is known to be a major oncogenic protein. The invention clearly demonstrates that the down-regulation of EZH2 by inhibiting the non-canonical NF-κB pathway restores the senescence program in melanoma cells. The invention provides evidence that EZH2 is a target gene for the non-canonical NF-κB pathway. Thus, isolation of the p52 specific binding sites within the EZH2 promoter as well as a particular peptide derived from p52 binding to the EZH2 promoter, as shown for the first time by the invention, provides a powerful tool for using such sequences and peptides as competitors for inhibiting p52 induced expression of EZH2, and thereby inducing senescence in cancer cells. Moreover, the inventors show that silencing of p52 is sufficient to inhibit EZH2 transcription and enable melanoma cells to enter into senescence, disregarding their mutation status on B-RAF and the level of activation of the MEK-Erk pathway. Finally, the effects of NF-κB2 silencing may be reproduced by inhibiting the kinase activating NF-κB2, called NIK

### Nucleic acids and compositions comprising thereof

Thus, a first aspect of the invention relates to an isolated nucleic acid sequence derived from the histone-lysine N-methyltransferase EZH2 promoter region. The nucleic acid sequence of the invention comprises at least one p52 binding site.

As used herein, the term "EZH2" refers to the catalytic subunit of polycomb repressive complex 2 (PRC2), which is a highly conserved histone methyltransferase that targets lysine-27 of histone H3 of several tumor-suppressor and anti-metastatic genes, repressing their transcription. EZH2 is an oncogenic protein that promotes tumor growth and also suppresses senescence in cancer such as in melanoma cells. The naturally occurring human EZH2 gene has a nucleotide sequence as shown in Genbank Accession number NM_004456 and the naturally occurring human EZH2 protein has an aminoacid sequence of 751 amino acids as shown in GenBank database under accession number NP_004447.

As used herein, the term "promoter" refers to a regulatory region of DNA generally located upstream a gene and can have regulatory elements several kilobases away from the transcriptional start site (enhancers). Many eukaryotic promoters contain a TATA box (sequence TATAAA), which in turn binds a TATA binding protein which assists in the formation of the RNA polymerase transcriptional complex. The TATA box typically lies very close to the transcriptional start site (often within 50 bases).

These p52 binding sites are comprised within a fragment of the EZH2 promoter region. It should be noted that the whole EZH2 promoter region presented by GenBank Accession no. NC_000007.13 and is shown as follows (and is represented by SEQ ID NO: 1):

AAAAAGTAGTAACGGGTCCGGCGGCAGCGCGGGCCGGGCGAGCGTCTCCGG
CAAACGCGGCGCCACAGCTGAGCCGACCTCCGGGCCCGCGCCCTCCCCTCCCCG
GGCACCACTAGGAGCGGCCAGCCCGGGCCTCGGCTCCGCGCGGGGAAACGAG
CGCGGCGGTTAAAACCGTTACCACCCCCGAGTTTTGAACTGGTTCAAACTTGGCT
TCCAGCACCCGCCCCGCCCCTCCCCCGCCCGGGAACTCTGCGGCGCCGGTTCCCG
CCAAGAGCCGCCGGCGCTTCGTCCCGCCCTTCGGCCGGTTCCCGCCACCTATCCTC
CCCGCCTCCCGTCCGCGGCGGGCTCCGGGCCCCCGCGATGTCTCCCGGTCCCCGC
GTGCCTGCACACCGCCTTCCTGAGAGGCGCCGTGTGTTCAGCGAAAGAACAAAG
AGACGGCGGCGGCGCTTCCACACGGCCAGTGGCGTCCCTTACAGCGAACCCCGCC
GCCGCCCGCGCGCACGCGCTGCCAGTGCCCGCCCGCCCACGAGCCCTGAGCGC
ACTCTGCGTGGGGCTGGCTCGGCGCCTCCGAGCCCGGCGGGCCCTGTGATTGGAC
GGGCGCCCGCCTCGCGTCCCGCCAATCGGGGCGGCGCTTGATTGGGCTGGGGGG
GCCAAATAAAAGCGATGGCGATTGGGCTGCCGCGTTTGGCGCTCGGTCCGGTCGC
GTCCGACACCCGGTGGGACTCAGAAGGCAGTGGAGCCCCGGCGGCGGCGGC
GGCGCGCGGGGGCGACGCGCGGGAACAACGCGAGTCGGCGCGCGGGACGAAGG
TAACGCGCCGCTGCGGGCGGCCCGGCCGGCGGGGCTCCGGGAGTGCGAACCGGG
CGGCGGCGGCGGCGCCAGGACCTCCCCGCCACTGCTGTGCCGGTCCCGGGTATCG
CCGAGCGGGGCTCACCGGGGCGCCGCGTTTGTAGGCGTGCGGGGGGTGGAGGG
TGAGGGGAGAGCCCCCCTCCCCGGAAGGAGCTGTGAGCTTCG

In one embodiment, the invention relates to a nucleic acid sequence comprising a p52 binding site derived from the EZH2 promoter region. It should be noted that each of these sequences is a fragment of the nucleic acid sequence as denoted by SEQ ID NO: 1.

More particularly, the p52 binding sites comprised within a fragment of the nucleic acid sequence of the EZH2 promoter may comprise sequences derived from either the positive strand or the negative strand. It should be noted that both sequences encompass nucleotide sequence located from nucleotide base at position -93 to nucleotide base at position -83 and comprise the first p52 binding site which is also referred to as EZH2-p52 binding site (1) as well as nucleotide sequence located from nucleotide base at position -34 to nucleotide base at position -24 and comprise the second p52 binding site which is also referred to as EZH2-p52 binding site (2) as represented as follows:
Specific binding of p52 to two EZH2-p52 sites within the EZH2 promoter:
EZH2-p52 binding site (1) EZH2-p52 binding site (2)
   **-1023 (-94) GGGGCTCACC (-83) (-34) GGAGAGCCC (-24) +1**

In one embodiment, the nucleic acid sequence of the invention comprises the nucleic acid sequence of a fragment of the EZH2 promoter (also referred as EZH2-p52 binding site 1), this fragment has the sequence shown as follows GGGGCTCACC (and represented by SEQ ID NO: 2) or a derivative thereof.

In one particular embodiment, the nucleic acid sequence of the invention consists of the sequence represented by SEQ ID NO: 2.

As used herein, the term "nucleic acid" refers to polynucleotides such as deoxyribonucleic acid (DNA), and ribonucleic acid (RNA). The terms should also be understood to include, as equivalents, analogs of either RNA or DNA made from nucleotide analogs, and, as applicable to the embodiment being described, single-stranded (such as sense or antisense) and double-stranded polynucleotides.

Preferably, the nucleic acid sequence of the invention is a sequence of at most 20 nucleotides. Thus, the nucleic acid sequence of the invention may have 10, 11, 12, 13, 14, 15, 16, 17, 18, 19 or 20 nucleotides.

It should be noted that the term "isolated" as also used herein with respect to nucleic acids, such as DNA or RNA, refers to molecules separated from other DNAs, or RNAs, respectively, which are present in the natural source of the macromolecule.

It should be appreciated that any of the p52 binding sites of the invention may comprise sequences derived from either the positive strand of the EZH2 promoter region, or the negative strand. A DNA single strand is indicated herein as "sense" or "positive" or "forward" strand, if an RNA version of the same sequence is translated or translatable into protein. A "negative strand" is its complementary "antisense" or "reverse" strand.

Therefore, in one embodiment, the nucleic acid sequence of the invention comprises the complementary sequence of SEQ ID NO: 2, shown as follows CCCCGAGTGG (and represented by SEQ ID NO: 3) or a derivative thereof.

In one particular embodiment, the nucleic acid sequence of the invention consists of the sequence represented by SEQ ID NO: 3.

In another embodiment, the nucleic acid sequence of the invention comprises the nucleic acid sequence of a fragment of the EZH2 promoter (also referred as EZH2-p52 binding site 2), this fragment has the sequence shown as follows GGAGAGCCC (and represented by SEQ ID NO: 4) or a derivative thereof.

In one particular embodiment, the nucleic acid sequence of the invention consists of the sequence represented by SEQ ID NO: 4.

In still another particular embodiment, the nucleic acid sequence of the invention comprises the complementary sequence of SEQ ID NO: 4, shown as follows CCTCTCGGG (and represented by SEQ ID NO: 5) or a derivative thereof.

In one particular embodiment, the nucleic acid sequence of the invention consists of the sequence represented by SEQ ID NO: 5.

By "derivative" is meant the "fragments", "variants", "analogs" of said nucleic acid sequence. A "fragment" of a molecule, such as any of the nucleic acid sequences of the present invention, is meant to refer to any nucleotide subset of the molecule. A "variant" of such molecule is meant to refer to a naturally occurring molecule substantially similar to either the entire molecule or a fragment thereof. An "analog" of a molecule can be a homologous molecule from the same species or from different species. The nucleic acid sequence of an analog or derivative may differ from the original sequence, when at least one base pair is deleted, inserted or substituted.

Typically, the invention encompasses derivatives of the nucleic acid sequences of the invention (SEQ ID NO: 2, 3, 4 and 5) which exhibits at least one, preferably all, of the biological activities of the reference nucleic acid sequence, provided the derivative retains the capacity of inhibiting EZH2 expression and/or inducing the senescence of cancer cells.

The skilled in the art can easily determine whether a peptide function-conservative variant. To check whether the newly generated sequences inhibit the expression of EZH2, and/or induce the senescence of cancer cells in the same way than the initially characterized sequence, a promoter activity and reporter gene assay (see in Example), a senescence associated-β-Gal assay may be performed with each sequence.

A second aspect of the invention relates to a construct comprising a nucleic acid sequence of at least one p52 binding site derived from the EZH2 promoter region.

In one embodiment, the construct of the invention comprises any of the nucleic acid sequences described above.

In one embodiment, the nucleic acid construct includes any of nucleic acid of the invention (e.g., SEQ ID NO: 2, 3, 4 or 5) operably linked to at least one promoter for directing transcription of nucleic acid in a cell. Any suitable promoter sequence can be used by the nucleic acid construct of the invention. Preferably the promoter is a constitutive promoter, a tissue-specific, or an inducible promoter.

Any of the constructs of the invention may further comprise operably linked reporter gene. As a non-limiting example, such reporter genes may be selected from the group consisting of luciferase, green fluorescent protein (GFP) or other fluorescent proteins, secreted alkaline phosphatase (SEAP) and β-galactosidase (β-gal).

In one embodiment, the constructs of the invention may be comprised within an expression vector or an expression vehicle.

As used herein, the term "expression vector", encompass vectors such as plasmids, viruses, bacteriophage, integratable DNA fragments, and other vehicles, which enable the integration of DNA fragments into the genome of the host. Expression vectors are typically self-replicating DNA or RNA constructs containing the desired gene or its fragments, and operably linked genetic control elements that are recognized in a suitable host cell and effect expression of the desired genes. These control elements are capable of effecting expression within a suitable host. Generally, the genetic control elements can include a prokaryotic promoter system or a eukaryotic promoter expression control system. Such system typically includes a transcriptional promoter, an optional operator to control the onset of transcription, transcription enhancers to elevate the level of RNA expression, a sequence that encodes a suitable ribosome binding site, RNA splice junctions, sequences that terminate transcription and translation and so forth. Expression vectors usually contain an origin of replication that allows the vector to replicate independently of the host cell.

Plasmids are the most commonly used form of vector but other forms of vectors which serves an equivalent function and which are, or become, known in the art are suitable for use herein. See, e.g., Pouwels et al. Cloning Vectors: a Laboratory Manual (1985 and supplements), Elsevier, N.Y.; and Rodriquez, et al. (eds.) Vectors: a Survey of Molecular Cloning Vectors and their Uses, Buttersworth, Boston, Mass (1988), which are incorporated herein by reference.

The term "operably linked" is used herein for indicating that a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein-coding regions, in the same reading frame.

It should be further appreciated that the invention further encompasses any host cell transfected or transformed with any of the constructs of the invention. "Cells", "host cells" or "recombinant host cells" are terms used interchangeably herein. It is understood that such terms refer not only to the particular subject cells but to the progeny or potential progeny of such a cell. Because certain modification may occur in succeeding generation due to either mutation or environmental influences, such progeny may not, in fact, be identical to the parent cell, but are still included within the scope of the term as used herein. "Host cell" as used herein refers to cells which can be recombinantly transformed with vectors constructed using recombinant DNA techniques. A drug resistance or other selectable marker is intended in part to facilitate the selection of the transformants. Additionally, the presence of a selectable marker, such as drug resistance marker may be of use in keeping contaminating microorganisms from multiplying in the culture medium. Such a pure culture of the transformed host cell would be obtained by culturing the cells under conditions which require the induced phenotype for survival.

The novel EZH2-p52 binding site of the invention provides a powerful tool for controlling the expression of genes harboring said sequence in their regulatory regions. More particularly, this site enables inhibition of the expression of EZH2. One possible way of interfering with the p52 activity mediated by the elements of the invention, is to use oligonucleotides (ODNs) comprising the nucleic acid sequences of these p52 binding sites as competitors of the endogenous sequence within the EZH2 promoter, for binding to p52. cis DNA elements (decoys) are short double stranded DNA oligonucleotides, able to bind their target DNA binding proteins. The decoys according to the present invention are replicas of EZH2-p52 binding sites. Like the natural elements, the decoys are able to bind p52. Therefore, when these elements are in surplus they will decoy p52 away from natural genomic elements. When regulatory factors, p52 in particular, are prevented from binding their target sequences, their regulatory effects on gene expression are generally impeded. The decoy strategy aims at providing an intracellular surplus of artificial DNA-binding sites for p52, thus sequestering this factor from its natural site(s). In this way, transcription enhancement from the natural sites is prevented.

Thus, a third aspect of the invention relates to a p52 decoy in the form of an oligonucleotide (ODN) comprising a nucleic acid sequence comprising at least one p52 binding site derived from the EZH2 promoter region.

In one embodiment, the ODN of the invention comprises any of the nucleic acid sequences described above.

In a particular embodiment, the decoy molecules of the invention are short, preferably, double stranded DNA modified ODNs, able to bind their target DNA binding p52. Accordingly, the decoy molecule of the invention comprises at least one repeat of the sequence of the invention, also designated EZH2-p52 binding sites (1) and (2) as denoted by SEQ ID NO: 2 and 4, or any derivative thereof as well as of its complementary sequences SEQ ID: 3 and 5, or any derivative thereof.

Preferably, the oligonucleotide of the invention is an oligonucleotide with most 20 nucleotides. Thus, the oligonucleotides of the invention may have 10, 11, 12, 13, 14, 15, 16, 17, 18, 19 or 20 nucleotides.

In a preferred embodiment, the decoy molecule of the invention, also referred as TFD1 is a double stranded DNA ODN comprising the following EZH2-p52 (2) forward sequence: ATCGGGTCTCC, as denoted by SEQ ID NO: 6, and the following EZH2-p52 (2) reverse sequence: GGAGAGCCCGAT, as denoted by SEQ ID NO: 7.

In another preferred embodiment, the decoy molecule of the invention, also referred as TFD4 is a double stranded DNA ODN comprising the following EZH2-p52 (1) forward sequence ATCGGGGCTCACC, as denoted by SEQ ID NO: 8, and the following EZH2-p52 (1) reverse sequence GGTGAGCCCCGAT, as denoted by SEQ ID NO: 9.

It should be further noted that the ODN of the invention may comprise modified nucleotide base or modified ODN.

In one embodiment, the oligonucleotide comprised within the decoy of the invention may be selected from the group consisting of DNA, RNA, LNA, PNA, INA and mixtures thereof and hybrids thereof, as well as phosphorous atom modifications thereof.

When used in the present context, the terms "locked nucleic acid monomer", "locked nucleic acid residue", "LNA monomer" or "LNA residue" refer to a bicyclic nucleotide analogue. LNA monomers are described in inter alia WO 99/14226, WO 00/56746, WO 00/56748, WO 01/25248, WO 02/28875, WO 03/006475 and WO 03/095467. By INA is meant an intercalating nucleic acid in accordance with the teaching of WO 03/051901, WO 03/052132, WO 03/052133 and WO 03/052134 incorporated herein by reference. An INA is an oligonucleotide or oligonucleotide analogue comprising one or more intercalator pseudonucleotide (IPN) molecules. Peptide nucleic acid (PNA) is an artificially synthesized polymer similar to DNA or RNA and is used in biological research and medical treatments. PNA is not known to occur naturally.

DNA and RNA have a deoxyribose and ribose sugar backbone, respectively, whereas PNA's backbone is composed of repeating N-(2-aminoethyl)-glycine units linked by peptide bonds. The various purine and pyrimidine bases are linked to the backbone by methylene carbonyl bonds. PNAs are depicted like peptides, with the N-terminus at the first (left) position and the C-terminus at the right.

As shown in the section Examples, the use of the decoy molecules of the invention clearly resulted in reduction of EZH2 expression, and significantly enhanced senescence of melanoma cells.

More specifically, the decoy molecules of the invention therefore may lead to reduction, suppression, inhibition, attenuation, elimination, repression or weakening of the expression of EZH2 in about 10-100%, as compared to control. Thus, the decoy molecules of the invention may lead to reduction of about 60% of the expression EZH2.

Reduction of EZH2 expression leads to increase of the senescence of cancer cells, and thereby the decoy molecules of the invention are useful for treating cancer. Thus, the decoy molecules of the invention may lead to increase of the senescence of cancer cells of about 10-100%, more specifically, of about 60% or 70% of increase of senescence, as compared to a control.

Therefore, it should be noted that the invention encompasses the use of any of the decoy molecules of the invention and any compositions comprising thereof, for use in a method for reducing EZH2 expression in a patient in need thereof and thereby, for use in a method for inducing the senescence of cancer cells of said patient (as described below) and for use in a method for treating cancer.

According to a further aspect, the invention relates to a pharmaceutical composition comprising as an active ingredient at least one p52 decoy in the form of an oligonucleotide (ODN) comprising a nucleic acid sequence of at least one p52 binding site derived from the EZH2 promoter region and a pharmaceutically acceptable carrier.

As used herein "pharmaceutically acceptable carrier" includes any and all solvents, dispersion media, coatings, antibacterial and antifungal agents and the like. The use of such media and agents for pharmaceutical active substances is well known in the art. Except as any conventional media or agent is incompatible with the active ingredient, its use in the therapeutic composition is contemplated.

The carrier can be solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

As clearly shown by the following Examples, the region located between 0 and -100 of the EZH2 promoter contains the binding sites for p52 as previously described. Thereby, binding of p52 in said EZH2 promoter region, results in enhanced expression of the EZH2 gene leading to tumor growth by inhibiting tumor differentiation, enhancing cell cycle progression, cell invasion, cancer stem cell self-renewal and angiogenesis.

The invention thus relates to a pharmaceutical composition comprising as an active ingredient at least one p52 decoy in the form of ODN comprising a nucleic acid sequence of at least one p52 binding site derived from the EZH2 promoter region and a pharmaceutically acceptable carrier for use in a method for treating cancer.

Preferably, said cancer is a cancer associated with the overexpression of EZH2. Examples of cancer over-expressing EZH2 include, for example, melanoma, esophageal cancer, lung cancer, osteosarcoma, colorectal cancer, renal cell carcinoma, bladder cancer, breast cancer, cholangiocellular carcinoma, chronic myelocytic leukemia (CML), acute myeloblastic leukemia (AML). In a particular embodiment, the cancer is melanoma.

The invention also relates to a method for treating cancer in a patient in need thereof comprising a step of administering to said patient a therapeutically effective amount of a p52 decoy in the form of an ODN comprising a nucleic acid sequence of at least one p52 binding site derived from the EZH2 promoter region as defined by the invention.

The term "therapeutically effective amount" is intended to mean that amount of a drug that will elicit the biological or medical response of a patient that is being sought by a clinician. Pharmaceutical compositions may be administered in any conventional dosage formulation. Pharmaceutical compositions typically comprise at least one active ingredient, as defined above, together with one or more pharmaceutical acceptable excipient.

It should be further noted that reduced expression of EZH2 and increased senescence in cancer cells may lead to an increase of the immunogenicity of cancers cells leading to an increase of efficacy of immunomodulatory compound.

In one embodiment, the method of treating cancer further comprises the step of administering to the patient a therapeutically effective amount of an additional therapeutic agent such as an immunomodulatory compound.

In a particular embodiment, the immunomodulatory compound is an immunostimulatory agent (e.g. a CTLA-4 antagonist, a PD-1 antagonist, or a PD-L1 antagonist as defined below).

### Peptides and compositions comprising thereof

Another aspect of the invention relates to an isolated peptide derived from the p52 polypeptide comprising or consisting of an amino acid sequence VQRKRRKAL (also referred as Peptide PI and represented by SEQ ID NO: 10) or a function-conservative variant thereof.

As used herein, the term "Peptide PI" relates to the peptide sequence of 9 amino acids corresponding to residues 336 to 344 of the p52 polypeptide (Uniprot accession number Q00653), which forms the p52 nuclear translocation signal, which is very specific. By way of example, Peptide PI has the sequence shown as follows VQRKRRKAL (represented by SEQ ID NO: 10). Thus, Peptide PI is considered as an inhibitor of the interaction between p52 and the promoter of the EZH2 gene by interfering with the localization of p52 in the nucleus.

In one embodiment, the peptide consists of the sequence SEQ ID NO: 10.

By an "isolated" peptide, it is intended that the peptide is not present within a living organism, e.g. within human body. However, the isolated peptide may be part of a composition or a kit. The isolated peptide is preferably purified. Such peptide is essentially free from contaminating cellular components, such as carbohydrate, lipid, or other proteinaceous impurities associated with the peptide in nature.

As used herein, the term "peptide" refers to an amino acid sequence having less than 25 amino acids. As used herein, the term "peptide" encompasses amino acid sequences having less than 25 amino acids, less than 20 amino acids, less than 15 amino acids, less than 10 amino acids. The term "peptide" does not exclude post-translational modifications that include but are not limited to phosphorylation, acetylation, glycosylation and the like. The term also applies to amino acid polymers in which one or more amino acid residue is an artificial chemical mimetic of a corresponding naturally occurring amino acid, as well as to naturally occurring amino acid polymers and non-naturally occurring amino acid polymer.

As used herein, the term "function-conservative variant" refers to a peptide in which a given amino acid residue in peptide has been changed (inserted, deleted or substituted) without altering the overall conformation and function of the peptide. Such variants include peptides having amino acid alterations such as deletions, insertions and/or substitutions. A "deletion" refers to the absence of one or more amino acids in the protein. An "insertion" refers to the addition of one or more of amino acids in the protein. A "substitution" refers to the replacement of one or more amino acids by another amino acid residue in the protein. Typically, a given amino acid is replaced by an amino acid having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 80 % as determined by BLAST or FASTA algorithms, more preferably at least 85%, still preferably at least 90 %, and even more preferably at least 95%, and which has the same or substantially similar properties or functions as the native or parent protein to which it is compared. Two amino acid sequences are "substantially homologous" or "substantially similar" when greater than 80 %, preferably greater than 85 %, preferably greater than 90 % of the amino acids are identical, or greater than about 90 %, preferably greater than 95 %, are similar (functionally identical) over the whole length of the shorter sequence. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

Typically, the invention encompasses peptides substantially identical to the Peptide PI in which one or more residues have been conservatively substituted with a functionally similar residue and which exhibits at least one, preferably all, of the biological activities of the reference peptide, provided the function-conservative variant retains the capacity of binding to the p52 binding sites derived from the EZH2 promoter region and/or reducing or inhibiting EZH2 expression, and/or inducing senescence of cancer cells.

The skilled in the art can easily determine whether a peptide is a function-conservative variant of P1. To check whether the newly generated peptides bind to the promoter of EZH2 and/or inhibit the expression of EZH2, and/or induce senescence of cancer cells in the same way than the initially characterized peptide, a promoter activity and reporter gene assay (see in Example), a quantitative RT-PCT analysis (see in Example), a senescence associated-β-Gal assay may be performed with each peptide. Additionally, a time-course and a dose-response performed *in vitro* or *in vivo* will determine the optimal conditions for each peptide.

Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid or another. The substitutions preferably correspond to conservative substitutions as indicated in the Table below:

| **Conservative substitutions** | **Type of Amino Acid** |
|---|---|
| Ala, Val, Leu, lle, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

In one embodiment, the peptides of the invention may comprise chemical modifications improving their stability and/or their biodisponibility. Such chemical modifications aim at obtaining polypeptides with increased protection of the polypeptides against enzymatic degradation *in vivo,* and/or increased capacity to cross membrane barriers, thus increasing its half-life and maintaining or improving its biological activity. Any chemical modification known in the art can be employed according to the present invention. Such chemical modifications include but are not limited to:
- replacement(s) of an amino acid with a modified and/or unusual amino acid, e.g. a replacement of an amino acid with an unusual amino acid like Nle, Nva or Orn; and/or
- modifications to the N-terminal and/or C-terminal ends of the peptides such as e.g. N-terminal acylation (preferably acetylation) or desamination, or modification of the C-terminal carboxyl group into an amide or an alcohol group;
- modifications at the amide bond between two amino acids: acylation (preferably acetylation) or alkylation (preferably methylation) at the nitrogen atom or the alpha carbon of the amide bond linking two amino acids;
- modifications at the alpha carbon of the amide bond linking two amino acids such as e.g. acylation (preferably acetylation) or alkylation (preferably methylation) at the alpha carbon of the amide bond linking two amino acids.
- chirality changes such as e.g. replacement of one or more naturally occurring amino acids (L enantiomer) with the corresponding D-enantiomers;
- retro-inversions in which one or more naturally-occurring amino acids (L-enantiomer) are replaced with the corresponding D-enantiomers, together with an inversion of the amino acid chain (from the C-terminal end to the N-terminal end);
- azapeptides, in which one or more alpha carbons are replaced with nitrogen atoms; and/or
- betapeptides, in which the amino group of one or more amino acid is bonded to the β carbon rather than the α carbon.

Another strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG) has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomular filtration (e.g., less than 60 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes. Such linkers may be used in modifying the polypeptides described herein for therapeutic delivery.

In one embodiment, the peptides of the invention may be fused to a heterologous peptide (i.e. peptide derived from an unrelated protein).

In a particular embodiment, the heterologous peptide is a peptide capable of being internalized into a cell.

Such peptides capable of being internalized into a cell used herein have in their respective primary amino acid sequences (that is, over their entire length) at least 25%, preferably at least 30% positively charged amino acid residues. The term "positively charged amino acids" (herein also referred to as "basic amino acids"), as used herein, denotes the entirety of lysine (K), histidine (H), and arginine (R) residue present in a particular peptide. In particular embodiments, the peptides used herein comprise in their respective primary amino acid sequences at least 35%, at least 40%, at least 45%, at least 50%, at least 55%, or at least 60% positively charged amino acid residues.

The term "capable of being internalized into a cell", as used herein, refers to the ability of the peptides to pass cellular membranes (including inter alia the outer "limiting" cell membrane (also commonly referred to as "plasma membrane"), endosomal membranes, and membranes of the endoplasmatic reticulum) and/or to direct the passage of a given agent or cargo through these cellular membranes. Such passage through cellular membranes is herein also referred to as "cell penetration". Accordingly, peptides having said ability to pass through cellular membranes are herein referred to as "cell-penetrating peptides" (CPP) also called "protein transduction domains" (PTD), "membrane translocation sequences" (MTS) or "translocating peptides". In the context of the invention, any possible mechanism of internalization is envisaged including both energy-dependent (i.e. active) transport mechanisms (e.g., endocytosis) and energy-independent (i.e. passive) transport mechanism (e.g., diffusion). As used herein, the term "internalization" is to be understood as involving the localization of at least a part of the peptides that passed through the plasma cellular membrane into the cytoplasma (in contrast to localization in different cellular compartments such as vesicles, endosomes or in the nucleus).

In one particular embodiment, the cell-penetrating peptide is the HIV transactivator of transcription (TAT) peptide as shown as follows GRKKRRQRRRPQ (represented by SEQ ID NO: 11) and as described in Vives et al., J. Biol. Chem., 272, 16010-16017, 1997).

Other cell-penetrating peptides (such as hydrophilic CPPs and amphiphilic CPPs) useful in the invention include for instance Penetratin or Antennapedia PTD, SynB1, Transportan, Pep-1, MAP, Polyarginines RxN (4<N<17) and Polylysines KxN (4<N<17).

In one embodiment, the peptide capable of being internalized into a cell is fused to the N-terminus or the C-terminus of the peptide of the invention, directly or via a peptide spacer. This complex is produced by making a fusion in frame of a nucleotide sequence encoding the peptide of the invention to a nucleotide sequence encoding the peptide/protein cargo, and expressing the resulting chimeric gene using standard recombinant DNA techniques.

The peptides of the invention may be produced by any suitable means, as will be apparent to those of skill in the art. In order to produce sufficient amounts of a Peptide PI or functional equivalents thereof, for use in accordance with the invention, expression may conveniently be achieved by culturing under appropriate conditions recombinant host cells containing the polypeptide of the invention. Preferably, the polypeptide is produced by recombinant means, by expression from an encoding nucleic acid molecule. Systems for cloning and expression of a polypeptide in a variety of different host cells are well known.

When expressed in recombinant form, the polypeptide is preferably generated by expression from an encoding nucleic acid in a host cell. Any host cell may be used, depending upon the individual requirements of a particular system. Suitable host cells include bacteria mammalian cells, plant cells, yeast and baculovirus systems. Mammalian cell lines available in the art for expression of a heterologous polypeptide include Chinese hamster ovary cells, HeLa cells, baby hamster kidney cells and many others. Bacteria are also preferred hosts for the production of recombinant protein, due to the ease with which bacteria may be manipulated and grown. A common, preferred bacterial host is *E coli.*

Another aspect of the invention relates to a nucleic acid sequence encoding a peptide according to the invention.

Another aspect of the invention relates to an expression vector comprising a nucleic acid sequence encoding a peptide according to the invention as well as a host cell comprising such expression vector.

Still another aspect of the invention relates to a nucleic acid encoding a peptide of the invention for use in a method for treating cancer.

Another aspect of the invention relates to a pharmaceutical composition comprising as an active ingredient at least a peptide of the invention or a nucleic acid according to the invention or an expression vector according to the invention or a host cell according to the invention and a pharmaceutically acceptable carrier

Another aspect of the invention relates to a method for treating cancer in a patient in need thereof comprising a step of administering to said patient a therapeutically effective amount of a peptide of the invention or a nucleic acid according to the invention or an expression vector according to the invention or a host cell according to the invention.

In one embodiment, the method of treating cancer further comprises the step of administering simultaneously or subsequently to the patient a therapeutically effective amount of an additional therapeutic agent such as an immunomodulatory compound.

In a particular embodiment, the immunomodulatory compound is an immunostimulatory agent (e.g. a CTLA-4 antagonist, a PD-1 antagonist, or a PD-L1 antagonist as defined below).

In one embodiment, said nucleic acid encoding an amino acid sequence consisting of VQRKRRKAL (SEQ ID NO: 10).

### Therapeutic methods and uses

The present invention provides methods and compositions (such as pharmaceutical compositions) for inducing senescence in cancer cells.

In a second aspect, the invention relates to an inhibitor of non-canonical NF-κB pathway for use in a method for inducing senescence in cancer cells.

As used herein, the term "inducing senescence of cancer cells" refers to the ability of a compound to increase the propensity of tumor cells to undergo senescence. Senescent cancer cells are slow-growing and possess one or more of the following additional characteristics attributed to senescent cells: heterochromatin formation, changes in cell shape, increased senescence-associated β-galactosidase activity. Cellular senescence has been regarded as a solely cell intrinsic mechanism of tumor suppression, i.e. suppressing tumor development through the induction of a stable cell cycle arrest. Moreover, senescent cancer cells are also able to trigger an immune response, designated as "senescence surveillance" which is crucial for tumor suppression as described in Hoenicke L. et al., 2012. Factors secreted from senescent cells were shown to attract innate immune cells (macrophages, neutrophils and NK cells) which mediated the clearance of senescent tumor cells. Thus senescence inducing therapies may be used to trigger immune responses against tumors as described in Xue, W. et al., 2007.

As used herein, the terms "cancer cell" or "tumor cell" are used interchangeably and refer to the total population of cells derived from a tumor or a pre-cancerous lesion.

Cancer cells susceptible to undergo senescent cancer cells are selected among melanoma, breast cancer, prostate cancer, liver cancer, bladder cancer, lung cancer, colon cancer, colorectal cancer, gastrointestinal cancer, pancreatic cancer, cervical cancer, ovarian cancer, bladder cancer, kidney cancer and various types of head and neck cancer.

In one embodiment, the cancer cells contain a BRAF wildtype.

In another embodiment, the cancer cells contain a BRAF V600E mutation.

In still another embodiment, the cancer cells contain other BRAF mutation such as V600D, V600K, V600R, and V600L mutations.

In a particular embodiment, the cancer cells undergoing in senescence are melanoma cells. Such melanoma cells may contain a BRAF wildtype or a BRAF V600E mutation.

As used herein, the term "non-canonical NF-κB pathway" refers to the alternative NF-κB signaling that predominantly targets activation of the p52/RelB NF-κB complex. This pathway depends on the inducible processing of p100 (or NF-κB2), a molecule functioning as both the precursor of p52 and a RelB-specific inhibitor. A central signaling component of the non-canonical pathway is NF-κB-inducing kinase (NIK), which integrates signals from a subset of TNF receptor family members and activates a downstream kinase, IκB kinase-a (IKKα), for triggering p100 phosphorylation and processing. Thus, the processing of p100 serves to both generate p52 and induce the nuclear translocation of the RelB/p52 heterodimer.

As used herein, the term "inhibitor of the non-canonical NF-κB pathway" refers to compounds which inhibit signalling through NF-κB2/p100, as well as compounds which inhibit the expression of the NF-κB2 gene. They include compounds which inhibit the activity of NF-κB2, or by inhibiting NF-κB2 signalling by other mechanisms. Typically, inhibitors of non-canonical NF-κB2 pathway are NIK inhibitors.

In one embodiment, the inhibitor of the non-canonical NF-κB pathway is an inhibitor of NF-κB2 gene expression.

An "inhibitor of expression" refers to any natural or synthetic compound that has a biological effect to inhibit the expression of a gene. Therefore, an "inhibitor of NF-κB2 (p100) gene expression" denotes a natural or synthetic compound that has a biological effect to inhibit the expression of NF-κB2 gene.

In one embodiment of the invention, said inhibitor of NF-κB2 gene expression is an antisense oligonucleotide, a siRNA, a shRNA or a ribozyme.

Inhibitors of NF-κB2 gene expression for use in the invention may be based on antisense oligonucleotide constructs. Anti-sense oligonucleotides, including anti-sense RNA molecules and anti-sense DNA molecules, would act to directly block the translation of NF-κB2 mRNA by binding thereto and thus preventing protein translation or increasing mRNA degradation, thus decreasing the level of NF-κB2, and thus activity, in a cell. For example, antisense oligonucleotides of at least about 15 bases and complementary to unique regions of the mRNA transcript sequence encoding NF-κB2 can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). It should be further noted that antisense oligonucleotides may be modified with phosphorothioate to prevent their *in vivo* hydrolysis by nucleases. Such modifications are well known in the art.

Small inhibitory RNAs (siRNAs) can also function as inhibitors of NF-κB2 gene expression for use in the invention. NF-κB2 gene expression can be reduced by contacting the tumor, subject or cell with a small double stranded RNA (dsRNA), or a vector or construct causing the production of a small double stranded RNA, such that NF-κB2 gene expression is specifically inhibited (i.e. RNA interference or RNAi). Methods for selecting an appropriate dsRNA or dsRNA-encoding vector are well known in the art for genes whose sequence is known (e.g. see Tuschi, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; International Patent Nos. WO 01/36646, WO 99/32619, and WO 01/68836). Short hairpin RNAs (shRNAs) can also function as inhibitors of gene expression for use in the invention.

In one particular embodiment, the sequence of the siRNA targeting NF-κB2 is CCCAGGUCUGGAUGGUAUUAUUGAA represented by SEQ ID NO: 12.

In one particular embodiment, the sequence of the siRNA targeting NF-κB2 is UUCAAUAAUACCAUCCAGACCUGGG represented by SEQ ID NO: 13.

In one particular embodiment, the sequence of the siRNA targeting NF-κB2 is AACCCAGGTCTGGATGGTATT represented by SEQ ID NO: 14.

In one particular embodiment, the sequence of the siRNA targeting NF-κB2 is CTGGATGGTATTATTGAATAT represented by SEQ ID NO: 15.

In one particular embodiment, the sequence of the siRNA targeting NF-κB2 is CGGCGTTGTCAACCTCACCAA represented by SEQ ID NO: 16.

In one particular embodiment, the sequence of the siRNA targeting NF-κB2 is AAGGACATGACTGCCCAATTT represented by SEQ ID NO: 17.

In one particular embodiment, the sequence of the siRNA targeting NF-κB2 is CACGGGCAGACCAGTGTCATT represented by SEQ ID NO: 20.

In a preferred embodiment, a pool of siRNAs targeting NF-κB2 may be used.

Such pool may comprise at least 2 siRNAs selected from SEQ ID NO: 12, 13, 14, 15, 16, 17 and 20.

In one particular embodiment, the sequence of the shRNA targeting NF-κB2 is ATAAGATTTGAAATAGGTG represented by SEQ ID NO: 21.

In another particular embodiment, the sequence of the shRNA targeting NF-κB2 is TCAGTTGCAGAAACACTGT represented by SEQ ID NO: 22.

Ribozymes can also function as inhibitors of NF-κB2 gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of NF-κB2 mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Antisense oligonucleotides, siRNAs, shRNAs and ribozymes useful as inhibitors of NF-κB2 gene expression can be prepared by known methods. These include techniques for chemical synthesis such as, e.g., by solid phase phosphoramadite chemical synthesis. Alternatively, anti-sense RNA molecules can be generated by *in vitro* or *in vivo* transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides, siRNAs, shRNAs and ribozymes of the invention may be delivered *in vivo* alone or in association with a vector. In its broadest sense, a **"vector"** is any vehicle capable of facilitating the transfer of the antisense oligonucleotide, siRNA or ribozyme nucleic acid to the cells and preferably cells expressing cancer cells. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide, siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

In one embodiment, the inhibitor of the non-canonical NF-κB pathway is a NIK inhibitor.

As used herein, the term "NF-kappa B inducing kinase" (NIK, also known as MAP3K14) refers to a serine/threonine kinase which regulates NF-kappa B pathway activation. NIK phosphorylates IkappaB kinase (IKK) which leads to degradation of IkappaB proteins and activation of NF-kappa B transcription factors. NIK is also known to induce p100 processing by stimulating site specific phosphorylation and ubiquitination of this precursor protein [Molecular Cell, No. 7, pp. 401-409 (2001)].

As used herein, the term "NIK inhibitor" refers to compounds which inhibit NIK activity, as well as compounds which inhibit the expression of the NIK gene.

Examples of NIK inhibitors useful in the methods of the invention are 6-azaindole aminopyrimidine derivatives as disclosed in PCT Application No. WO2010/042337; alkynyl alcohol derivatives as disclosed in PCT Application No. WO2009/158011; pyrazoloisoquinoline derivatives as disclosed in PCT Application No WO 2005/012301 and WO2004/005287. It should be further noted that a cell-based assay useful for the screening and the identification of NIK inhibitors, i.e. candidate molecules inhibiting NIK activity is disclosed in PCT Application No. WO2013/01424.

In one embodiment, said NIK inhibitor is an inhibitor of NIK gene expression such as an antisense oligonucleotide, a siRNA, a shRNA or a ribozyme.

Inhibitors of NIK gene expression for use in the invention such as antisense oligonucleotides, siRNAs, shRNAs or ribozymes may be prepared by known methods as previously described for the inhibitors of NF-κB2 gene expression.

In one particular embodiment, the sequence of the siRNA targeting NIK is GCCAGUCCGAGAGUCUUGAUCAGAU represented by SEQ ID NO: 18.

In another particular embodiment, the sequence of the siRNA targeting NIK is AUCUGAUCAAGACUCUCGGACUGGC represented by SEQ ID NO: 19.

In a preferred embodiment, a pool of siRNAs targeting NIK may be used.

Such pool may comprise both siRNAs of SEQ ID NO: 18 and SEQ ID NO: 19.

In one embodiment, the inhibitor of the non-canonical NF-κB pathway is an inhibitor of NF-κB2 processing.

In a particular embodiment, the inhibitor of NF-κB2 processing is an inhibitor of the 26S proteasome. Typically, an inhibitor of the 26S proteasome is Bortezomib (marketed as Velcade® by Millennium Pharmaceuticals and used for the treatment of multiple myeloma) disclosed in the US Patent No. 5,780,454.

In another particular embodiment, the inhibitor of NF-κB2 processing is a GSK3α inhibitor as described in Bang et al., 2013. Typically, a GSK3α inhibitor is (N-(4-methoxybenzyl)-N'-(5-nitro-1,3-thiazol-2-yl)urea (AR-A014418).

In one embodiment, the inhibitor of the non-canonical NF-κB pathway is an inhibitor of the interaction of p52 and the promoter of the EZH2 gene.

As used herein, the terms "inhibitor of the interaction" means preventing or reducing the direct or indirect association of one or more molecules, nucleic acids, peptides, proteins or or peptidomimetics. As used herein, the term "inhibitor of the interaction between p52 and the promoter of the EZH2 gene" is a molecule which can prevent the interaction between p52 and the promoter of the EZH2 gene by competition or by fixing to one of the molecule.

In one embodiment, the inhibitor of the interaction of p52 and the promoter of the EZH2 gene comprise or consist of a nucleic acid sequence derived from the EZH2 promoter region, comprising a p52 binding site of the invention as described above.

In a particular embodiment, said nucleic acid sequence derived from the EZH2 promoter region is a p52 decoy in the form of an oligonucleotide (ODN) comprising a nucleic acid sequence represented by SEQ ID NO: 2, 3, 4 and 5 or any derivatives thereof.

In another embodiment, said nucleic acid sequence derived from the EZH2 promoter region is a p52 decoy in the form of an oligonucleotide (ODN) comprising a nucleic acid sequence represented by SEQ ID NO: 2, 3, 4 and 5 or any derivative thereof.

In a particular embodiment, the decoy molecule of the invention, also referred as TFD1, is a double stranded DNA ODN comprising the sequence ATCGGGTCTCC, as denoted by SEQ ID NO: 6, as well as of its complementary sequence GGAGAGCCCGAT, as denoted by SEQ ID NO: 7.

In another particular embodiment, the decoy molecule of the invention, also referred as TFD4, is a double stranded DNA ODN comprising the sequence ATCGGGGCTCACC, as denoted by SEQ ID NO: 8, as well as of its complementary sequence GGTGAGCCCCGAT, as denoted by SEQ ID NO: 9.

In another embodiment, the inhibitor of the interaction of p52 and the promoter of the EZH2 gene comprise or consist of a peptide sequence derived from p52, also referred as Peptide PI represented by SEQ ID NO: 10 or a function-conservative variant as described above.

In a third aspect, the invention relates to an inhibitor of the non-canonical NF-κB pathway for use in a method for increasing tumor immunogenicity.

As used herein, the term "immunogenicity" refers to the ability of a particular substance to provoke an immune response. Tumors are immunogenic and enhancing tumor immunogenicity aids in the clearance of the tumor cells by the immune response.

In one embodiment, the inhibitor of non-canonical NF-κB pathway is selected from the group consisting of an inhibitor of expression of NF-κB2 gene, a NIK inhibitor, an inhibitor of the interaction of p52 and the promoter of the EZH2 gene (e.g. a p52 decoy), an inhibitor of NF-κB2 processing (e.g. an inhibitor of the 26S proteasome) as disclosed above.

In another aspect, the invention also relates to an inhibitor of the non-canonical NF-κB pathway for use in a method for increasing efficacy of a therapy with an immunomodulatory compound.

As used herein, the term "enhancing the efficacy" refers to the increase of the number of patients affected with a cancer and treated with an immunomodulatory compound which exhibit a clinically beneficial response to said treatment.

As used herein, the "patient response" can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition (i.e. reduction, slowing down or complete stopping) of metastasis; (6) enhancement of anti-tumor immune response, which may, but does not have to, result in the regression or rejection of the tumor; (7) relief, to some extent, of one or more symptoms associated with the tumor; (8) increase in the length of survival following treatment; and/or (9) decreased mortality at a given point of time following treatment.

Preferably, said beneficial response is a long-term response. Accordingly, the term "long-term response" is used herein to refer to a complete response for at least 1 year, more preferably for at least 3 years, most preferably for at least 5 years following treatment.

Accordingly, in another aspect, the invention further relates to an inhibitor of the non-canonical NF-κB pathway for use in a method for improving the survival time of a patient affected with a cancer and treated with an immunomodulatory compound.

In one embodiment, the survival time is progression-free survival (PFS).

The term "Progression-Free Survival" (PFS) in the context of the invention refers to the length of time during and after treatment during which, according to the assessment of the treating physician or investigator, the patient's disease does not become worse, i.e., does not progress. As the skilled person will appreciate, a patient's progression-free survival is improved or enhanced if the patient experiences a longer length of time during which the disease does not progress as compared to the average or mean progression free survival time of a control group of similarly situated patients.

In one embodiment, the survival time is Overall Survival (OS).

The term "Overall Survival" (OS) in the context of the invention refers to the average survival of the patient within a patient group. As the skilled person will appreciate, a patient's overall survival is improved or enhanced, if the patient belongs to a subgroup of patients that has a statistically significant longer mean survival time as compared to another subgroup of patients. Improved overall survival may be evident in one or more subgroups of patients but not apparent when the patient population is analysed as a whole.

In one embodiment, the inhibitor of non-canonical NF-κB pathway is selected from the group consisting of an inhibitor of expression of NF-κB2 gene, a NIK inhibitor, an inhibitor of the interaction of p52 and the promoter of the EZH2 gene (e.g. a p52 decoy or a p52 peptide), an inhibitor of NF-κB2 processing (e.g. an inhibitor of the 26S proteasome) as disclosed above.

As used herein, the term "immunomodulatory compound" refers to a compound that modulates one or more of the components (e.g., immune cells, or subcellular factors, genes regulating immune components, cytokines, chemokines or such molecules) of a host's immune system. Preferably, the immunomodulatory compound is an immunostimulatory agent. Immunomodulatory agents may include, but are not limited to, small molecules, peptides, polypeptides, fusion proteins, antibodies. Immunomodulatory antibodies are a promising class of anti-cancer therapies, due to their ability to promote a broad and sustained anti-cancer immune response in cancer patients. Within the context of the invention, it shuld be further noted that the immunomodulatory compound is not an antigen (i.e. cancer antigen).

Typically, immunomodulatory compounds are CTLA-4 antagonist, PD-1 antagonist, or PD-L1 antagonist.

As used herein, the term "CTLA-4 antagonist" means any molecule that can interfere with the specific interaction of a CTLA-4 receptor (also called CD152) with its natural ligands B7.1 (also called CD80) and B7.2 (also called CD86). An antagonist can act as a competitive inhibitor or a noncompetitive inhibitor of CTLA-4 binding to its ligands. Such agents can include antibodies (including antigen binding portions thereof), peptides, and non-peptide small organic molecules, as well as substances (such as anti-sense or interfering RNA molecules) that decrease the expression of a CTLA-4 receptor. An anti-CTLA-4 antibody (or antigen binding portion thereof) that can interfere with the binding of CTLA-4 to its ligands B7.1 and/or B7.2 is one example of a CTLA-4 antagonist. Other antagonists are described herein, and/or will be readily apparent to those of skill in the art.

In a particular embodiment, the CTLA-4 antagonist is an anti-CTLA-4 antibody. Examples of anti-CTLA-4 antibodies include, but are not limited to, those described in the following PCT Application Nos. WO 2001 /014424, WO 2004/035607, the antibodies disclosed in U.S. Publication No. 2005/0201994, and the antibodies disclosed in granted European Patent No. EP1212422B1. Additional CTLA-4 antibodies are described in U.S. Patent Nos. 5,81 1 ,097, 5,855,887, 6,051 ,227, and 6,984,720; in PCT Publication Nos. WO 01 /14424 and WO 00/37504; and in U.S. Publication Nos. 2002/0039581 and 2002/086014. Other anti-CTLA-4 antibodies that can be used in a method of the present invention include, for example, those disclosed in: WO98/42752; U .S. Patent Nos. 6,682,736 and 6,207,156; Hurwitz et al., Proc. Natl. Acad. Sci. USA, 95(17):10067-10071 (1998); Camacho et al., J. Clin. Oncology, 22(145):Abstract No. 2505 (2004) (antibody CP-675206); Mokyr et al., Cancer Res, 58:5301 -5304 (1998), U.S. Patent Nos. 5,977,318; 6,682,736; 7,109,003, and

A specific example of an anti-CTLA-4 antibody useful in the methods of the invention is MDX-010 (also called BMS-734016 or Ipilimumab and marketed as Yervoy used for the treatment of melanoma) as disclosed in the PCT Application No. WO2001/014424.

As used herein, the term "PD-1 antagonist" means any molecule that attenuates inhibitory signal transduction mediated by the binding of PD-L1 (also called B7-H1 or CD274) to PD-1. In specific examples of the invention, a PD-1 antagonist is a molecule that inhibits, reduces, abolishes or otherwise reduces inhibitory signal transduction through the PD-1 receptor signalling pathway. Such decrease may result where: (i) the PD-1 antagonist of the invention binds to a PD-1 receptor without triggering signal transduction, to reduce or block inhibitory signal transduction mediated by PD-L1; (ii) the PD-1 antagonist binds to PD-L1, preventing its binding to PD-1; (iii) the PD-1 antagonist binds to, or otherwise inhibits the activity of, a molecule that is part of a regulatory chain that, when not inhibited, has the result of stimulating or otherwise facilitating PD-1 inhibitory signal transduction mediated by PD-L1; or (iv) the PD-1 antagonist inhibits PD-1 expression or PD-L1 expression, especially by reducing or abolishing expression of one or more genes encoding PD-1 or one PD-L1.

In a particular embodiment, the PD-1 antagonist is an antibody selected from the group consisting of anti-PD-1 antibodies or anti-PD-Ll antibodies. Examples of anti-PD-1 antibodies include, but are not limited to, those described in the following PCT patent applications Nos: WO2003/099196; WO2006/121168; WO2009/014708; WO004/004771; WO2004/072286 WO2004/056875; WO2008/083174; WO2007/005874 and WO/2009/073533). A specific example of an anti-PD-1 antibody is MDX-1106 (see Kosak, US 20070166281. Another specific example of anti-PDl antibody, is lambrolizumab (formerly MK-3475). Examples of anti-PD-Ll antibodies include, but are not limited to, those described in the following applications Nos: WO2006/133396; WO2008/083174 and US 2006/0110383. A specific example of an anti-PD-Ll antibody useful in the methods of the invention is MDX-1105 (WO2007/005874) which is a high-affinity, fully human, PD-L1-specific, IgG4 (S228P) monoclonal antibody that inhibits the binding of PD-L1 to both PD-1.

The invention further relates to a method for improving the survival time of a patient affected with a cancer and treated with an immunomodulatory compound comprising a step of administering simultaneously or subsequently a therapeutically effective amount of inhibitor of the non-canonical NF-κB pathway to said patient.

In another aspect, the invention further relates to an inhibitor of the non-canonical NF-κB pathway for use in a method for reducing or inhibiting expression of EZH2 in cancer cells.

Thus, the inhibitors of the non-canonical NF-κB pathway of the invention therefore may lead to reduction, suppression, inhibition, attenuation, elimination, repression or weakening of the expression of EZH2 in about 10-100% as compared to control. Thus, the inhibitors of the invention may lead to reduction of about 60% of the expression EZH2.

In one embodiment, the inhibitor of the non-canonical NF-κB pathway is selected from the group consisting of an inhibitor of expression of NF-κB2 gene, a NIK inhibitor, an inhibitor of the interaction of p52 and the promoter of the EZH2 gene (e.g. a p52 decoy or a p52 peptide), an inhibitor of NF-κB2 processing (e.g. an inhibitor of the 26S proteasome) as disclosed above.

Examples of cancer over-expressing EZH2 include, for example, melanoma, esophageal cancer, lung cancer, osteosarcoma, colorectal cancer, renal cell carcinoma, bladder cancer, breast cancer, cholangiocellular carcinoma, chronic myelocytic leukemia (CML), acute myeloblastic leukemia (AML). In a particular embodiment, the cancer is melanoma.

### Combination therapies of the invention:

The inhibitor of non-canonical NF-κB pathway of the invention may be administered to a patient with an appropriate additional therapeutic agent such as an immunomodulatory compound useful in the treatment of cancer from which the patient suffers or is susceptible to.

The invention also relates to a method for treating cancer in a patient in need thereof comprising a step of administering to said patient a therapeutically effective amount of an inhibitor of the non-canonical NF-κB pathway and an immunomodulatory compound.

As used herein, the term "patient in need thereof' refers to a patient having a cancer and preferably an epithelial cancer such as melanoma.

As used herein, the term "treating" refers to clinical intervention designed to alter the natural course of the patient or cell being treated during the course of clinical pathology. Desirable effects of treatment include decreasing the rate of disease progression, ameliorating or palliating the disease state, and remission or improved prognosis. For example, a patient is successfully "treated" if one or more symptoms associated with cancer are mitigated or eliminated, including, but are not limited to, reducing the proliferation of (or destroying) cancerous cells, decreasing symptoms resulting from the disease, increasing the quality of life of those suffering from the disease, decreasing the dose of other medications required to treat the disease, delaying the progression of the disease, and/or prolonging survival of patients.

The methods of this invention may find use in treating conditions where enhanced immunogenicity is desired such as increasing tumor immunogenicity for the treatment of cancer. A variety of cancers may be treated, or their progression may be delayed. In one embodiment, the patient has melanoma. The melanoma may be at early stage or at late stage.

The administration of the inhibitor of non-canonical NF-κB pathway of the invention and the immunomodulatory compound, (e.g., CTLA-4 antagonist or PD-1 antagonist) can be carried out simultaneously, e.g., as a single composition or as two or more distinct compositions using the same or different administration routes. Alternatively, or additionally, the administration can be done sequentially, in any order. Alternatively, or additionally, the steps can be performed as a combination of both sequentially and simultaneously, in any order. In certain embodiments, intervals ranging from minutes to days, to weeks to months, can be present between the administrations of the two or more compositions. For example, the additional therapeutic agent may be administered first, followed by inhibitor of non-canonical NF-κB pathway of the invention. However, simultaneous administration or administration of the inhibitor of non-canonical NF-κB pathway of the invention first is also contemplated.

In one embodiment, the inhibitor of non-canonical NF-κB pathway is selected from the group consisting of an inhibitor of expression of NF-κB2 gene, a NIK inhibitor, an inhibitor of the interaction of p52 and the promoter of the EZH2 gene (e.g. a p52 decoy), an inhibitor of NF-κB2 processing (e.g. an inhibitor of the 26S proteasome) as disclosed above.

Accordingly, in one aspect, the invention relates to a pharmaceutical composition comprising an inhibitor of the non-canonical NF-κB pathway according to the invention and an immunomodulatory compound.

In another aspect, the invention relates to a kit-of-part composition comprising an inhibitor of the non-canonical NF-κB pathway according to the invention and an immunomodulatory compound.

The terms "kit", "product" or "combined preparation", as used herein, define especially a "kit-of-parts" in the sense that the combination partners as defined above can be dosed independently or by use of different fixed combinations with distinguished amounts of the combination partners, i.e. simultaneously or at different time points. The parts of the kit of parts can then, e.g., be administered simultaneously or chronologically staggered, that is at different time points and with equal or different time intervals for any part of the kit of parts. The ratio of the total amounts of the combination partners to be administered in the combined preparation can be varied. The combination partners can be administered by the same route or by different routes. When the administration is sequential, the first partner may be for instance administered 1, 2, 3, 4, 5, 6, 12, 18 or 24 h before the second partner.

In still another aspect, the invention relates to a pharmaceutical composition or a kit-of-part composition for use in a method for treating cancer comprising an inhibitor of the non-canonical NF-κB pathway according to the invention and an immunomodulatory compound.

The invention will be further illustrated by the following figures and examples. However, these examples and figures should not be interpreted in any way as limiting the scope of the present invention.

### FIGURES:

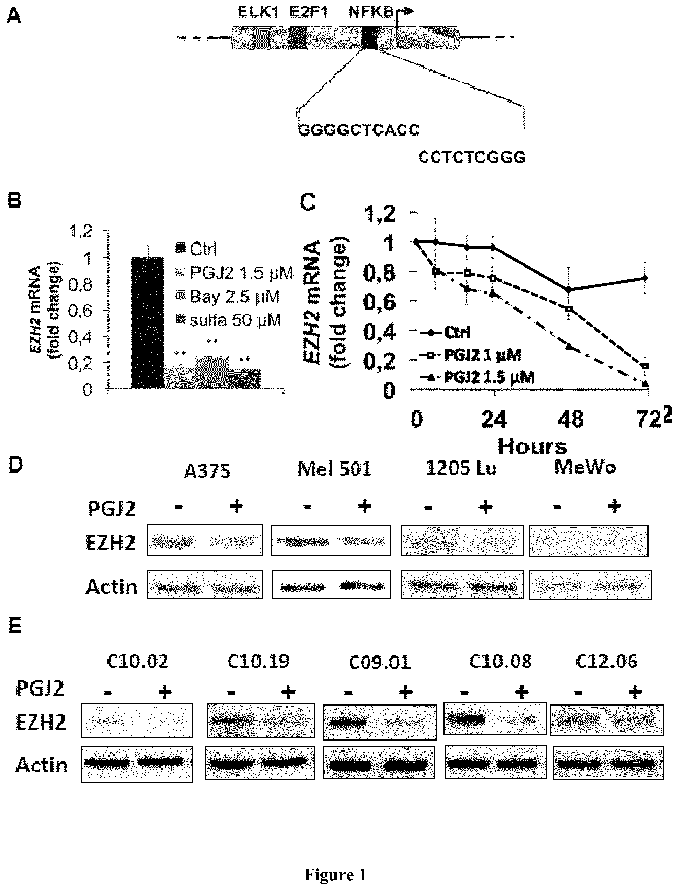

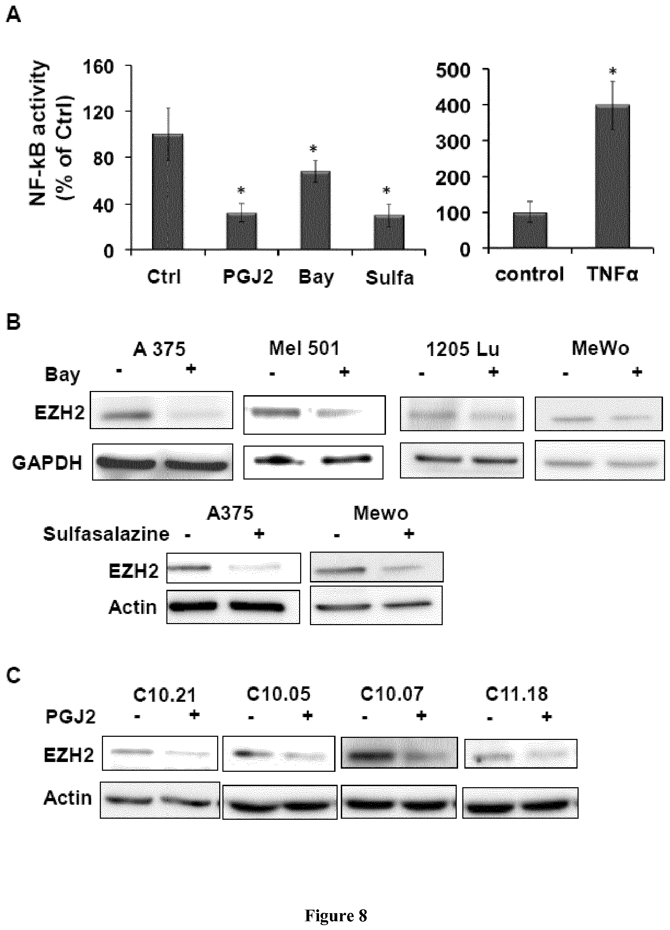

**Figure 1****: NF-kB inhibitors down-regulate EZH2.** (**A**) Schematic representation of the terminal portion of the EZH2 promoter with underlined positions of ELK1, E2F1 and NF-kB (localize at-93/-83 and -34/-24) binding sites and their sequence. (**B**) Quantitative polymerase chain reaction with reverse transcription (qRT-PCR) analysis of EZH2 mRNA prepared from A375 cells treated for 3 days with PGJ2, Bay 11-7082 and sulfasalazine. (**C**) Time and dose dependent effect of PGJ2 on EZH2 mRNA level in A375 cells using qRT-PCR analysis. For B and C, SB34 mRNA was used for normalization. *P <0.05 and **P<0.01, compared to control (Student t test). (**D**) EZH2 protein level determined by western blot (with actin as a loading control) in melanoma cell lines treated with PGJ2 at 2µM for 3 days. (**E**) EZH2 protein level after 3 days treatment with PGJ2 at 2µM in melanoma cells cultured from patients' metastases. See also Figure 8.

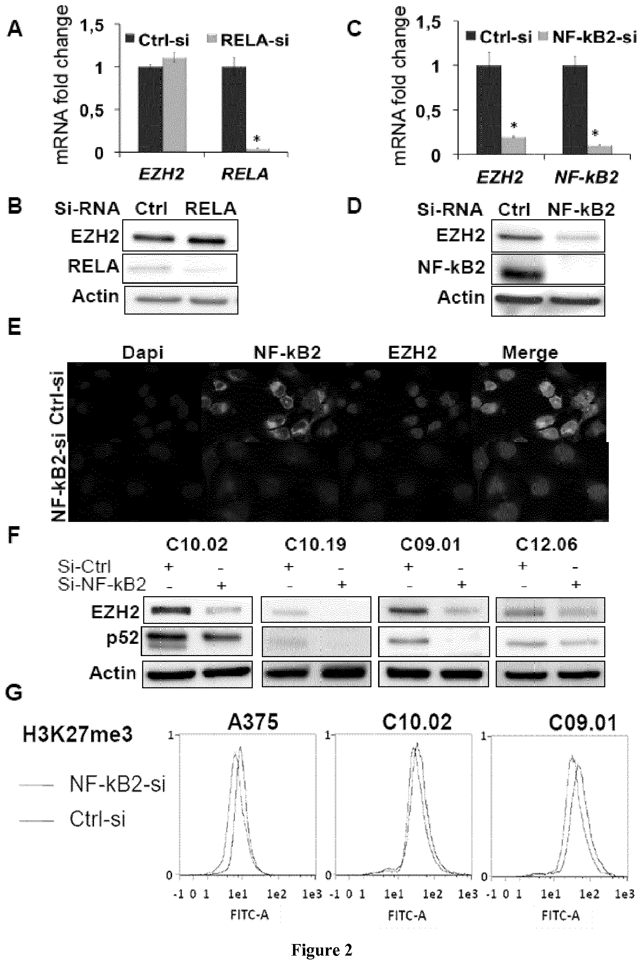

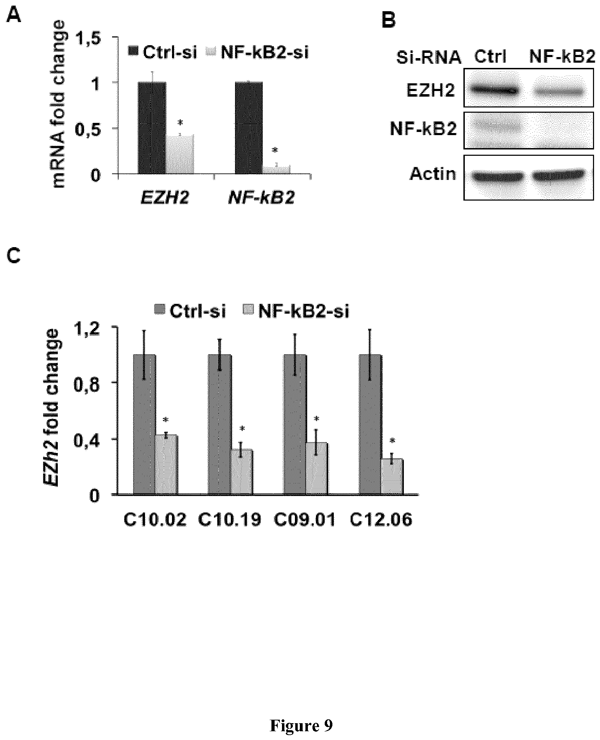

**Figure 2****: NF-kB2 silencing down-regulates EZH2.** (**A**), EZH2 mRNA fold change assessed by qRT-PCR 3 days post transfection with SiRNAs targeting RELA in A375 cells. SB34 mRNA was used for normalization. *P <0.05, compared to control (Student t test). (**B**), EZH2 and RelA protein levels 3 days post transfection. (**C**), EZH2 mRNA fold change 3 days post transfection with SiRNAs targeting NF-kB2/P100 in A375 cells. (**D**), EZH2 and p52 protein level 3 days post transfection. (**E**), p100/p52 and EZH2 immunofluorescent staining in A375 cells transfected with NF-kB2 siRNA. 40x magnification. Dapi counter stains nuclei. (**F**), EZH2 and p52 protein levels 3 days post transfection of melanoma cells cultured from patients' samples. (**G**), Flow cytometry analysis of total level of Histone 3 Lysine 27 trimethyl (H3K27me3) in A375 and melanoma cells from patient samples 4 days post NF-kB2-siRNA transfection. See also Figure 9.

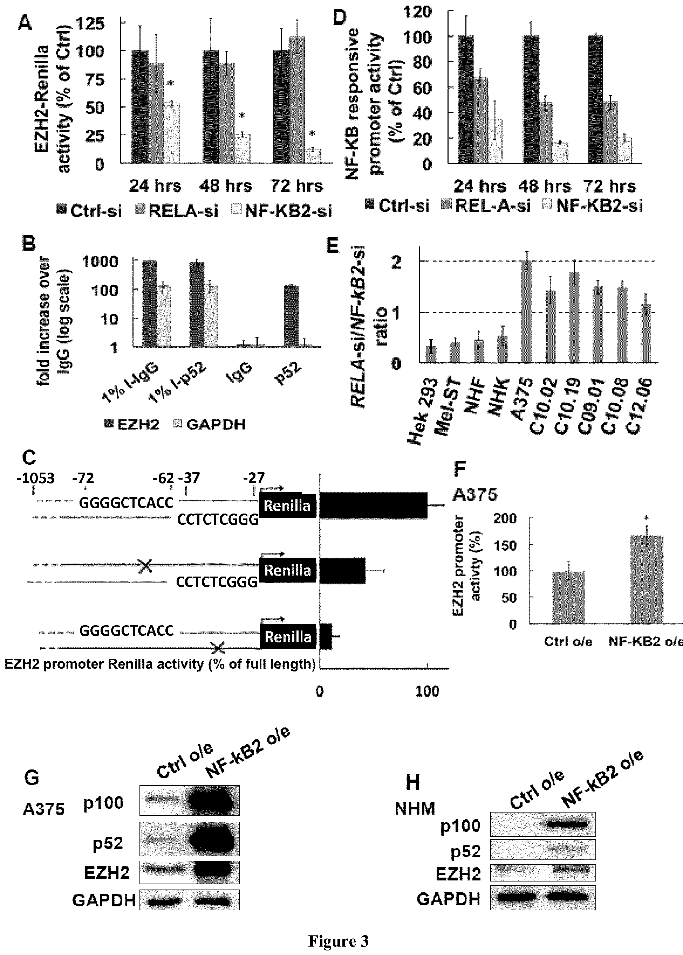

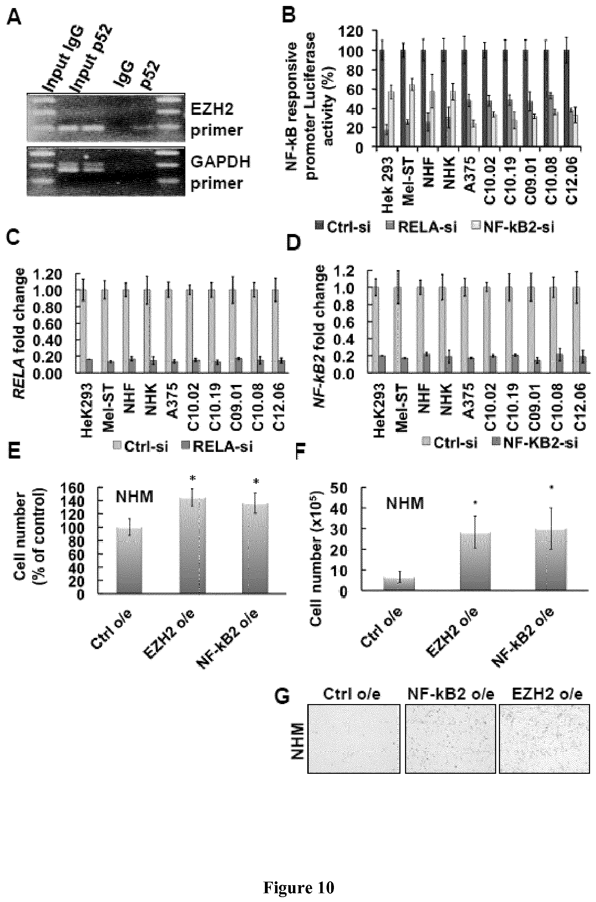

**Figure 3****: Direct regulation of EZH2 by NF-kB2 non canonical pathway that contributes in majority to total NF-kB activity in melanoma cells.** (**A**) photoluminescence measurement of EZH2 promoter-Renilla construct (EZH2-Renilla) activity in A375 cells at times indicated post transfection with Si-RNA against RELA or NF-kB2. Scrambled RNA was used as a control. (**B**) CHIP quantification of EZH2 promoter immunoprecipitated with p100/p52 antibody. The ratio immunoprecipitated/input was 0.146%. (**C**) Photoluminescence activity of EZH2 promoter-Renilla construct (EZH2-Renilla) after deletion of putative binding sites for p52 (-93/-83 bps from the transcription binding site for Delp52-1 and -34/-24 for Delp52-2) compared to the original non-deleted EZh2-Renilla construct. EZH2 constructs were transfected in A375 cells grown under standard culture conditions for 3 days post-transfection. (**D**) Photoluminescence quantification of luciferase activity of NF-kB responsive promoter luciferase construct after silencing of RELA or NF-kB2. (**E**) Calculated ratio between luciferase activity in RELA versus NF-kB2-silenced cells. Non tumoral cells (HEK293, Mel-ST which are immortalized primary human melanocytes (Gupta et al., 2005), Normal Human Keratinocytes (NHK), Normal Human Fibroblast (NHF)) are used as a control as compared to melanoma cell line (A375) and melanoma cells cultured from melanoma patients' metastases. (**F**) EZH2-Renilla activity measured by photoluminescence 3 days post infection of A375 cells with an adenovirus expressing NF-kB2 or vector control (Ctrl). (**G**) Western blot analysis of indicated proteins in A375 overexpressing NF-kB2 or vector control (Ctrl). (**H**) Western blot analysis of indicated proteins in normal human melanocytes (NHM) 3 days after infection with an adenovirus expressing NF-kB2 or vector control (Ctrl). See also Figure 10.

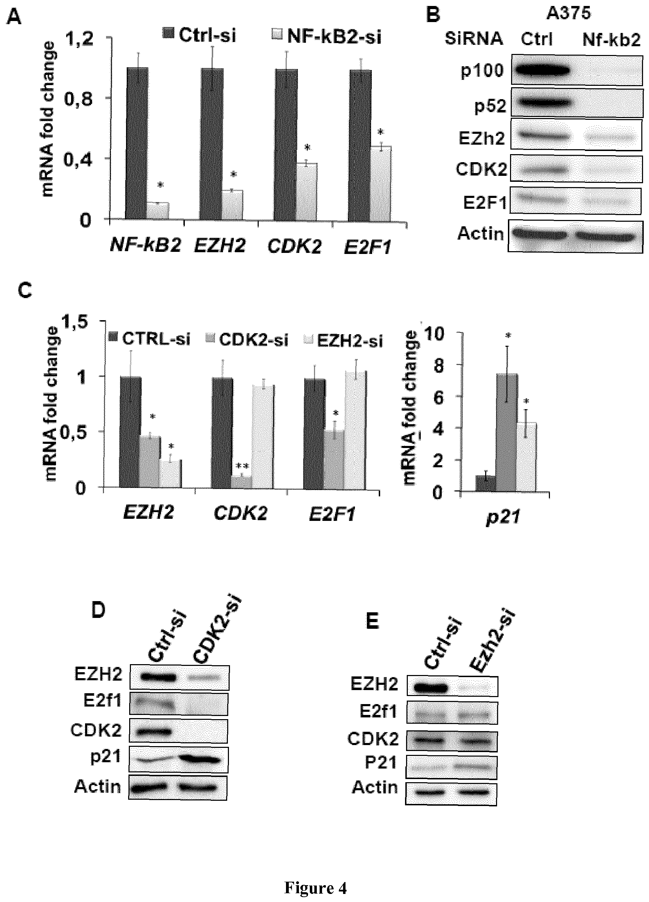

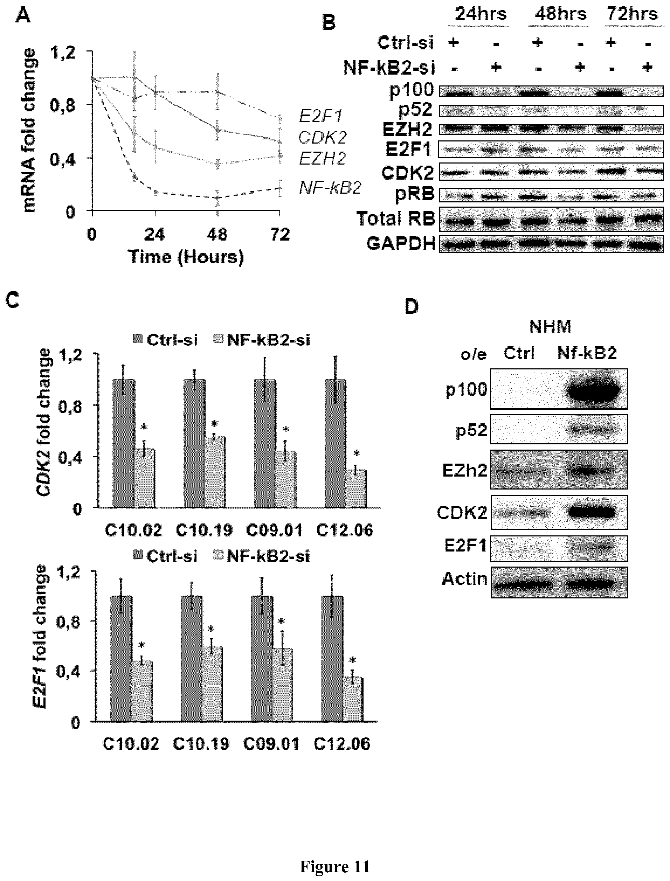

**Figure 4****: NF-kB2 silencing down-regulates CDK2 and E2F1.** (**A**) qRT-PCR analysis of mRNA of NF-kB2 (p100), EZH2, CDK2 and E2F1 in A375 cells 4 days post-transfection with SiRNA against NF-kB2. (**B**) Western blot analysis of related protein levels 4 days post-transfection of A375 melanoma cells with NF-kB2-SiRNA. Actin was used as a loading control. (**C**) Effect of CDK2 and EZH2 silencing using RNA interferences on mRNA level of cell cycle regulators in A375 (3 days post transfection). (**D, E**) Western blot analysis of the cell cycle regulators 3 days post transfection of A375 cells with CDK2-siRNA (**D**) or EZH2-siRNA (E). See also Figure 11.

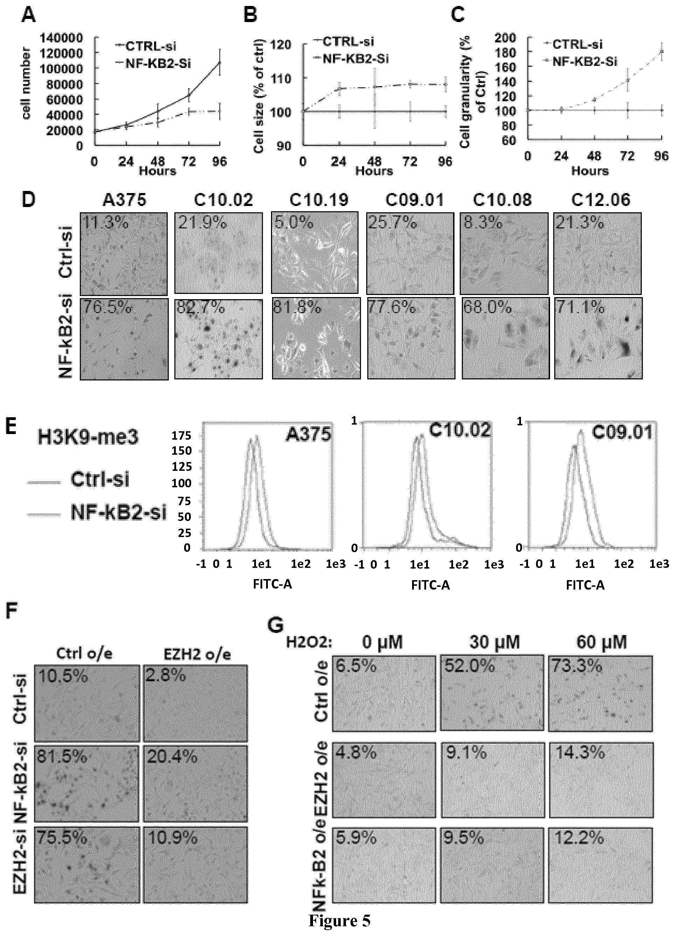

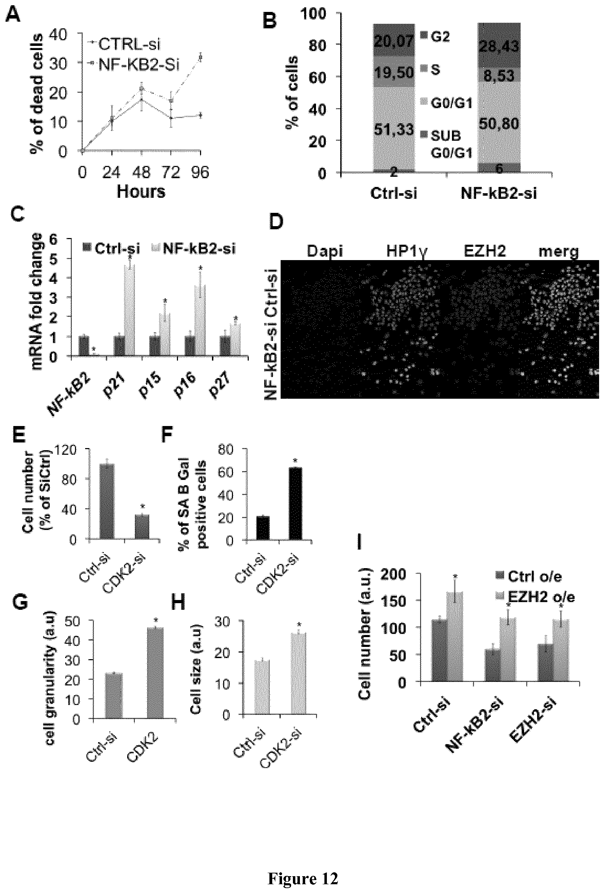

**Figure 5****: Senescence induction by NF-kB2 silencing.** A375 melanoma cells were transfected with NF-kB2-siRNA and analyzed for proliferation and phenotypic changes at times indicated. (**A**) Cell number determination of adherent cells. (**B-**C) Flow cytometry analysis of cell size (**B**) and granularity (**C**). (**D**) SA-β-Gal staining of melanoma cell lines and cells cultured from patients tumors. (**E**) Flow cytometry analysis of total H3K9me3 level in melanoma cells. (**F**) EZH2 overexpression compared with vector control prevents senescence induction by NF-kB2 or EZH2-silencing in A375 melanoma cells. Representative images of SA-β-Gal staining. (**G**) EZH2 overexpression compared with vector control prevents senescence induced by H2O2 in NHM. SA-β-Gal staining was performed after 4 days treatment with H2O2. Numbers of SA-β-Gal positive cells are indicated in upper left corner of each image. See also Figure 12.

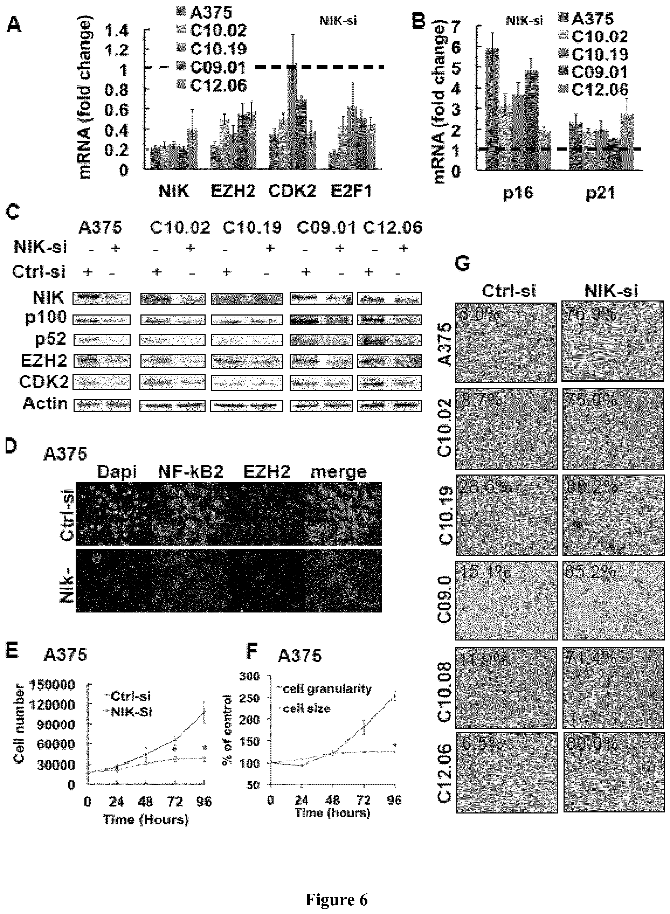

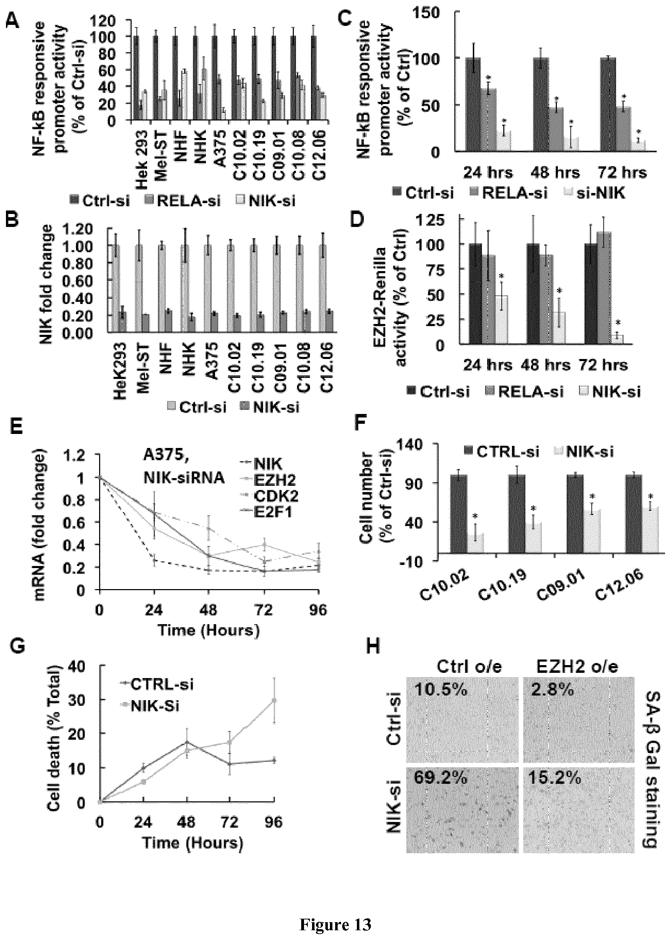

**Figure 6****: NF-kB inducing kinase (NIK)-silencing down-regulates EZH2 and induces senescence. (A and B)** qRT-PCR analysis of mRNA from A375 cells and melanoma cells from patients 4 days post-transfection with SiRNA directed against NIK, showing decreased levels of EZH2, CDK2 and E2F1 (A), and increased levels of p21 and p16 (B). SB34 was used as a control for normalization. Results are reported as fold change compared to scrambled siRNA. * indicates P <0.05, compared to control (Student t test). (C), Proteins from melanoma cells (A375 and cells cultured from patients' tumors) transfected with siRNA against NIK were harvested 4 days post-transfection and immunoblotted for indicated targets. (D), Immunocytofluorescent staining of p52 and EZH2 in A375 silenced for NIK showing positive correlation between NIK and p52 expressions. (E), Time course analysis of the effect of NIK-siRNA on A375 cell number. (F and G) Increase of cell size and granularity analyzed by flow cytometry after NIK silencing in A375 cells. Percentage of control (scrambled RNA) is represented. (H), SA-β-Gal staining in A375 and melanoma cells from patients 4 days post NIK-silencing. Numbers of SA-β-Gal positive cells are indicated in upper left corner of each image. See also Figure 13.

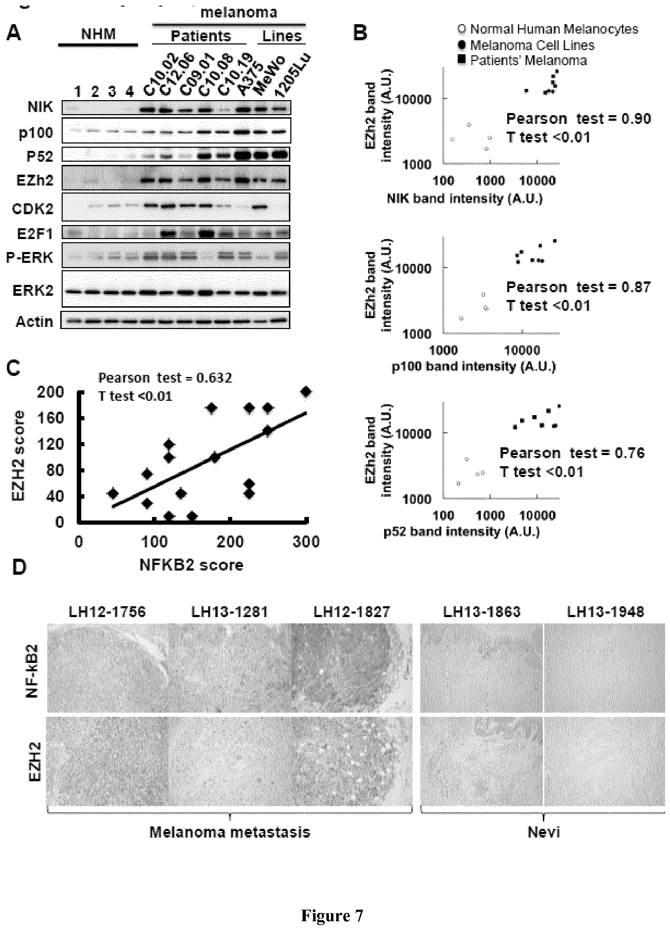

**Figure 7****: Correlation between EZH2 and proteins from the non-canonical NF-kB pathway.** (**A**) Western blot analysis in NHM (1-4), melanoma cell lines and melanoma cells extracted from patients (metastases). (**B**) Scatter plot of intensity values for protein expression obtained in A, EZH2 protein bands are plotted against NIK, p100 and p52. Data analysis (see also Figure 76): Pearson test indicates good correlation between EZH2 and NIK (0.90), EZH2 and p100 (0.87), EZH2 and p52 (0.76). (**C**), Immunohistostaining of EZH2 and NF-kB2 in 16 metastatic melanoma from patients compared with 5 nevi (see also Table S2). Two slides from each specimen were stained alternatively with NF-KB2 antibody or EZH2 antibody indicating increased co-expression of NF-KB2 and EZH2 in metastatic melanomas versus nevi. Samples were stained with DAB (dark-brown) and were counterstained with DAPI (blue). Representative images of samples listed in Table S2 are depicted in C. (**D**), Correlation analysis of immunohistostaining of NF-kB2 and EZH2 in 16 metastatic melanoma tumors. Pearson test was used for correlation of EZH2 with NF-kB2.
**Figure 8****: NF-kB inhibitors down-regulate EZH2.** (**A**), NF-kB luciferase activity measured by photoluminescence in A375 after 4 days treatment with PGJ2 (1.5 µM), Bay 11-7082 (2 µM) and sulfasalazine (50 µM) or TNFα (10 µM) as a positive control. *P <0.05, compared to control (Student t test). (**B**), Western blot analysis of EZH2 level in melanoma cells treated 4 days with Bay 11-7082 (2 µM) or sulfasalazine. GAPDH or Actin were used as loading control. (**C**), EZH2 protein level 4 days post PGJ2 treatment (1.5 µM) in melanoma cells isolated from patients. Actin was used as a loading control.
**Figure 9****: NF-kB2 silencing down-regulates EZH2.** (**A**), *EZH2* mRNA level analyzed by qRT-PCR 3 days post transfection with NF-kB2-siRNA in Mel501 melanoma cell line. *SB34* mRNA was used for normalization. (**B**), EZH2 protein level 3 days post transfection with NF-kB2-siRNA in Mel501 cells. (**C**), *EZH2* mRNA level analyzed by qRT-PCR 3 days post transfection with NF-kB2-siRNA in melanoma cells isolated from patients. **P* <0.05, compared to control (Student t test).
**Figure 10****: Direct regulation of EZH2 by NF-kB2 non canonical pathway that contributes in majority to total NF-kB activity in melanoma cells.** (**A**) Agarose gel loaded with samples derived from PCR amplification of CHIP experiments performed with an antibody directed against NF-kB2 (p100/p52). Amplification of immunoprecipitated DNA using primers directed against *EZH2* promoter in the samples containing antibody against NF-kB2 indicates the interaction of p52 with the promoter of *EZH2. GAPDH* primers were used as negative control. **(B)** NF-kB promoter activity determined by photoluminescence assay 3 days post transfection with SiRNA directed against RELA or NF-kB2 followed by transfection with NF-kB luciferase reporter plasmid (transfection rate was normalized by β-Gal reporter plasmid). Scrambled siRNA was used as a control for siRNA transfection. Non-cancer cells (HEK293, Mel-ST, Normal Human Keratinocytes (NHK), Normal Human Fibroblast (NHF) are used as a control as compared to melanoma cell line (A375) and melanoma cells cultured from melanoma patients' metastases. Results are reported as percentage of control. (**C, D**) Test for efficiency of silencing of *RELA* and *NF-kB2* with targeting siRNAs in all cells used in (**B**) by qRT-PCR. SB34 was used as a control for normalization. Results are reported as fold change compared to scrambled siRNA. (E) Cell number determination of normal human melanocytes (NHM) 3 days post infection with an adenovirus expressing NF-kB2 or EZH2 or empty vector used as a control. Results are expressed as percentage of control. (**F, G**) 4 days post infection with NF-kB2 or EZH2 adenovirus, NHM were detached and seeded at a very low density (20 000 cells) in a 6 well plate for 6 days. Representative pictures taken at 10 x magnification after Cristal violet staining of the cells are depicted (**G**). Cell count averaged of 3 independent assays (G). * indicates *P* <0.05, compared to control (Student t test).
**Figure 11****: Regulation of EZH2, CDK2 and E2F1 by the non canonical pathway. (A)** Time course analysis by qRT-PCR of mRNA coding for EZH2, CDK2, E2F1 in A375 cells transfected with RNA interference targeting NF-kB2 or scrambled RNA (Ctrl-si). **(B)** Time course analysis of indicated proteins by Western blot in A375. (**C, D**) qRT-PCR analysis of mRNA of *CDK2* (C) and *E2F1* (D) in melanoma cells isolated from patients 4 days post-transfection with SiRNA against NF-kB2. SB34 was used as a control for normalization. Results are reported as fold change compared to scrambled siRNA. (E) Western blot analysis showing increased protein level of EZH2 and CDK2 in normal human melanocytes (NHM) 3 days post infection with an adenovirus overexpressing NF-kB2. Actin was used as a loading control. * indicates *P* <0.05, compared to control (Student t test).
**Figure 12****: NF-kB2 silencing induces senescence.** (**A**) Percentage of dead cells induced by NF-kB2 silencing in A375 cells. Culture supernatant and adherent cells were collected for flow cytometry analysis after propidium Iodide (PI) staining as a marker of cell death. (**B**) Cell cycle analysis using PI staining in A375 cells 4 days post NF-kB2-siRNA transfection. (**C**) mRNA fold change *of p21, p15, p16* and *p27* analyzed by qRT-PCR in A375 cells 4 days post transfection with NF-kB2-siRNA. * indicates *P* <0.05, compared to control (Student t test). (**D**) Immunofluorescence staining of A375 cells showing the inverse correlation between HP1γ and EZH2 4 days post transfection with NF-kB2-siRNA. Dapi was used to counterstain nuclei. (**E**) A375 cells were transfected with CDK2-siRNA for 4 days and analyzed by flow cytometry. Cell number analysis, values are represented as percentage of control ± SD. (**F**) SA-β-Gal positive cells analyzed by fluorimetric measurement of C12FDG cleavage. After 1 hour incubation with 100nM bafilomycin, cells were incubated for 1 hour with C12FDG whose cleavage was then quantified by measurement of C12-fluorescein fluorescence intensity. (**G and H**) Cell granularity and cell size. (**I**) A375 cells were infected with an adenovirus overexpressing EZH2 (EZH2 o/e) under CMV promoter or adenovirus containing an empty vector (Ctrl o/e) 24 hours prior to transfection with NF-kB2-or EZH2-siRNA for 3 days. Representation of total cell number (average of 5 independent experiments). **P* <0.05, compared to control (Student t test).
**Figure 13****. Effect of NIK-silencing on total NF-kB activity in melanoma cells and on senescence induction through EZH2 downregulation.** (**A**) NF-kB sensitive promoter activity assessed by photoluminescence analysis of luciferase activity in various melanoma cells transfected with RNA interference targeting NIK, RELA, or scrambled siRNA prior to transfection with a NF-kB sensitive promoter expressing Luciferase. (**B**) Test for efficiency of silencing of *NIK* with targeting siRNA in all cells used in (A) by qRT-PCR. SB34 was used as a control for normalization. Results are reported as fold change compared to scrambled siRNA. (C) Time-dependant effect of NIK or RELA silencing on NF-kB sensitive promoter activity in A375 cells to compare the contribution of NIK signaling versus the classic NF-kB pathway to total NF-kB activity. (**D**) EZH2 promoter activity assessed by photoluminescence analysis of renilla activity. A375 cells were transfected with siRNA targeting NIK, RELA or scrambled siRNA 24 hours prior to be transfected with EZH2 promoter-Renilla construct. (**E**) Time course qRT-PCR analysis of *NIK, EZH2, CDK2, E2F1* mRNAs from A375 cells transfected with SiRNA against NIK. SB34 was used as a control for normalization. Results are reported as fold change compared to scrambled siRNA. * indicates *P* <0.05, compared to control (Student t test). (**F**) NIK repression reduces cell number. Cell number analysis 4 days post transfection with NIK-siRNA in melanoma cells isolated from patients. (**G**) Percentage of dead cells analyzed by flow cytometry in A375 cells transfected with NIK-siRNA (PI positive cells). (**H**) EZH2 overexpression prevents senescence induced by NIK silencing. A375 cells were infected with adenovirus overexpressing EZH2 (EZH2 o/e) or with empty vector (Ctrl o/e) for 8hrs. 24 hours post infection, cells were transfected with NIK-siRNA. 3 days post transfection, cells were stained for SA-β-gal activity to determine senescence. Mean numbers of SA-β-Gal positive cells are indicated in upper left corner of each image.

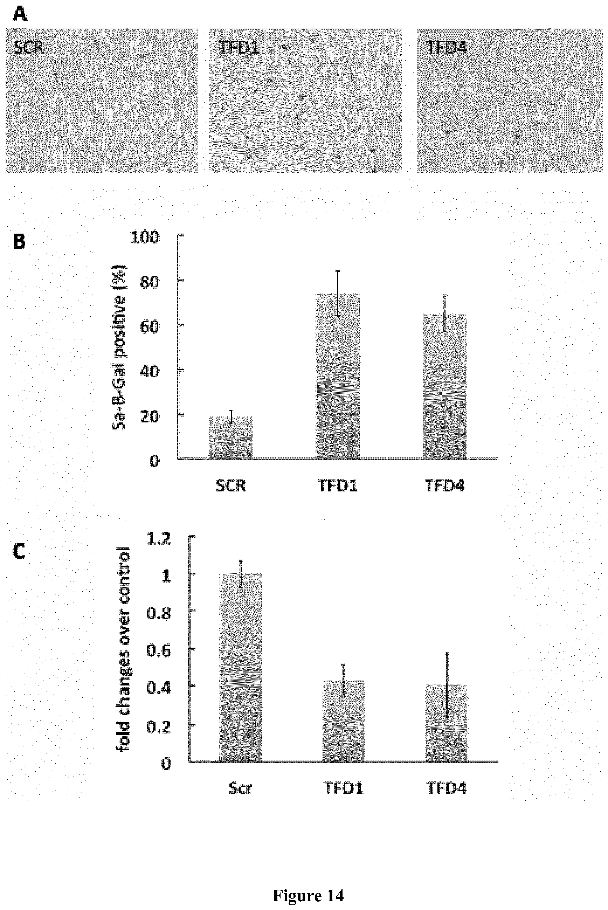

**Figure 14****: Transcription factor decoys (TFD) for NFKB2 can decrease EZH2 levels and induce senescence in melanoma.** (**A**) Representative images from a SA-β-Gal staining of A375 transfected for four days with TFDs, blue staining indicates senescence. A scramble sequence was used as control. (**B**) Quantification of the levels of senescence induced by TFDs as described in A, values are provided in percent of the total cell numbers. (C) Q-PCR assays of mRNA levels of EZH2 in A375 cells transfected with TFDs for four days. Sb34 was used for normalization.

### EXAMPLE 1: INHIBITION OF THE NON-CANONICAL NF-κB PATHWAY SIGNALLING INHIBITS EXPRESSION OF EZH2 IN CANCER CELLS LEADING THEM UNDERGOING SENESCENCE

### Material & Methods

**Cell Culture:** HEK293, Human A375 (CRL-1619), SK-Mel-28 (HTB-72) and MeWo (HTB-65) melanoma cell lines were purchased from American Tissue Culture Collection (Molsheim, France). Human primary melanoma cells were extracted from metastatic melanoma specimens that were obtained from the Department of Dermatology and the Biobank of the University hospital of Nice, France. All patients provided informed written consent. Biopsy was dissected and digested for 1-2 h with collagenase A (0.33 U/ml), dispase (0.85 U/ml) and Dnase I (144 U/ml) with rapid shaking at 37°C. Large debris were removed by filtration through a 70-µm cell strainer. Viable cells were obtained by Ficoll gradient centrifugation. All melanoma cells were cultured in RPMI medium or DMEM (Gibco Life Science, Life Technologies, Grand Island, NY), supplemented with 7% FBS and penicillin-streptomycin 50 µM (Gibco) at 37°C in 5% CO2. For each experiment, cells were cultured with their optimal medium supplemented with 2% FBS. Mel-ST cells were a kind gift from the laboratory of Prof. Bob Weimberg. Normal human melanocytes (NHM), keratinocytes (NHK) and fibroblast (NHF) were extracted from fresh human foreskin according to previous protocol (Larribere et al., 2004) and cultured in Media 254 containing supplements for melanocytes (S-002-5, Gibco Life Science), Epilife medium (Life technologies) supplemented with Human Keratinocytes Growth Supplement (HKGS, Life technologies) or DMEM medium supplemented with FGF mix.

**Transfection experiments with Small interfering RNAs (siRNAs):** Typically 30 000 to 50 000 cells were plated in 6-well culture plates and transfected 24 hours later with 25nM or 50nM siRNAs using Lipofectamine RNAimax (Invitrogen) according to manufacturer protocol. If not otherwise described cells were incubated with the si-RNA for 4 days prior harvesting for further experiments. Gene solution si-RNA against NF-kB2 (1027416, Qiagen, Germantown MD) and stealth siRNA (NFKB2HSS107146, Invitrogen) were used to silence NF-kB2, both giving similar results, Stealth si-RNA against MAP3K14/NIK (MAP3K14VHS40827, Invitrogen) and RELA/p65 si-RNA (FlexiTube S102663094, Quiagen). As non specific control, a scramble sequence siRNA was used (cat. 16106, Ambion, Life Technologies, Grand Island, NY).

**EZH2 and NF-kB2 overexpression:** Recombinant adenovirus carrying CMV-controlled NF-kB2 or EZH2 coding sequences (#1534 and #1371 Vector Biolabs, Philadelphia, PA) were amplified in HEK293 for 5 days and isolated by centrifugation at 24,000 rpm for 30min. Virus preparations were aliquoted and stored at -80°C until use. For virus infection, cells were plated overnight and mock infected or infected with NF-kB2 or EZH2 overexpressing virus sat no more than 5 PFU/cells. The concentration of infectious viral particles was determined before infection by PFU determination using progressive dilution. At 8 hours post infection, cells were washed 3 times with PBS and cultured in their medium supplemented with 7% FBS.

**Promoter activity and Reporter Gene Assays:** Cells were cotransfected withEZH2 promoter Renilla luciferase reporter construct (switchgear genomics, Menlo Park, CA) using CMV promoter b-Galactosidase construct as a control of transfection efficiency and Lipofectamine 2000 (Invitrogen) as transfecting agent. Typically cells were harvested, if not otherwise specified, 72hrs post transfection and luciferase activity test was carried on according to manufacturer protocol (Promega,Madison, Wi).

Cells were transfected with NF-kB-luciferase reporter (NF-kB-Luc, Qiagen Germantown MD) sensitive to overall NF-kB activation and with CMV promoter b-Galactosidase (pCMVβGal) for normalization purpose. Lipofectamine 2000 (Invitrogen, Carlsbad, CA) was used as transfection agent. 48 hrs post transfection, luciferase activity was tested using the luciferase assay system kit (Promega) and measured with the Clarity Luminescence Microplate Reader (Bio-Tek Instruments, Winooski, VE).

For gene reporter activity post gene silencing using RNA interference, cells were first transfected with SiRNAs according to experimental design using as transfecting agent Lipofectamine RNAimax as previously described. 24 hrs post transfection cells were again transfected using EZH2 promoter Renilla construct or NF-kB-luciferase reporter and pCMVβGal. 48 hrs after addition of the reporter plasmid, cells were harvested and luciferase or Renilla activity measured as above mentioned.

**Determination of cell number:** Cells were counted using Malassez chamber. Average and standard deviation were calculated from triplicate experiments.

**Chemicals:** Bay 11-7082 (Calbiochem, EMD Millipore, Lyon), 15-deoxy-delta12,14-Prostaglandin J2 (Cayman, Ann Arbor, MI, USA), sulfasalazine and Hydrogen peroxide solution (Sigma, St Luis, MO), human recombinant TNFα (PerproTech, US).

**Senescence associated-β-Gal assay:** Cells were washed then fixed for 15 min at RT and stained using the Senescence β-Galactosidase staining kit following manufacturer instruction (Cell Signalling, Beverly, MA). A blue color reflects the β-Gal activity.

**Quantitative RT-PCR analysis:** Total RNA was isolated from cells using RNAeasy minikit (Qiagen) according to manufacturer's procedure. Reverse transcription was performed using AMV reverse transcription system (Promega) while quantitative PCR was performed with Power Sybr green (Applied biosystem, Life Technologies, Grand Island, NY) following manufacturer instructions (List of primers are available in supplementary information). PCR reaction was carried on a Step One plus Real-Time PCR system (Applied Biosystems). All analyses were done in triplicate and melting curve analysis was performed to control for product quality and specificity. Expression levels were calculated using the comparative method of relative quantification, with SB34 as normalizer. Data were analyzed for their statistical significance applying Student's t-test. Results are presented as mean±SEM relative to the control.

**Western Blot:** Twenty to thirty micrograms of total proteins were run on 12% SDS-polyacrylamide gel electrophoresis. After transfer to polyvinylidene fluoride membrane (Millipore), immunoblotting was performed according to Laemli method. Glyceraldehyde-3-phosphate dehydrogenase (GAPDH), β-Actin or ERK2 were used as loading controls. Membranes were then incubated with an enhanced chemiluminescence system (Amersham) and immunoreactive bands were visualized using the luminescent image analyzer Fujifilm LAS-4000 (GE Healthcare).

**Mutagenesis and cloning:** EZH2 promoter was obtained from swissgeargenomics (cat# 1072), mutations of p52 binding site was performed with quickchange XLII kit (Agilent, Les Ulis) following manufacturer instructions. List of primers used and sequence analysis are available below.

**H3K9me3 and H3K27me3 determination by flow cytometry:** Cells were harvested in EDTA 2mM and fixed in 4% formaldehyde. After washing, antibodies against H3K9me3 (#5327, Cell signaling) or FITC conjugated anti-H3K27me3 (FCABS300A4, Millipore) were incubated for 1 hour on ice. Cells were then washed in PBS, and detection of H3K9me3 required a 30 min incubation on ice with a secondary antibody FITC-goat anti-rabbit IgG (Invitrogen) followed by washing. Analysis was performed with a MACSQuant Analyser (MyltenyiBiotec, Paris) cytometer.

Cell size and granularity were determined by flow cytometry using MACSQuant Analyser (MyltenyiBiotec, Paris) cytometer. FSC (forward scatter) and SSC (Side Scatter) were used to quantify the relative size and granulosity, respectively.

**Cell cycle analysis:** Cells were detached in PBS/EDTA 1 mM, fixed in 70% Ethanol overnight at -20°C, then incubated in PBS containing 20ug/ml RNAse A (Invitrogen) and 40 ug/ml of propidium iodide for 30 min prior to measurement with a MACS QuantAnalyser (Myltenyi Biotec, Paris). And data were analyzed using machine associated software.

**Immunofluorescence:** For immunofluorescence microscopy, cells cultured on coverslips were fixed in 4% formaldehyde then washed, incubated with 50 mM of ammonium chloride in PBS for 10 min at RT and permeabilized with 0.1% Triton X-100 for 2 min. After washing with PBS, cells were blocked for 1 hour in 5% goat serum. The following primary antibodies were used: mouse monoclonal antibody against EZH2 (AC22; Cell Signalling #3147), rabbit monoclonal antibody against NF-kB2 p100/p52 (18D10 Cell signaling #3017S), rabbit polyclonal antibody against HP1γ (Cell signaling #2619S). Incubation with primary antibodies was performed overnight at 4 degrees and then cells were washed with PBS. Secondary antibodies Alexa Fluor® 488 goat anti-rabbit (Invitrogen, #A11034, 1:1000) and Alexa Fluor® 568 goat anti-mouse (Invitrogen, #A110-31, 1:1000) and Dapi (1mg/L) were then applied for 60 minutes at RT, followed by final wash in PBS. Coverslips were mounted in Fluoromount-G (Interchim, FP-483331). Confocal images were acquired on LSM-510 Meta (Carl Zeiss Microimaging Inc., Germany) equipped with Plan-Apochromat 40x/1.4 oil immersion objective, ZEN2009 software (Carl Zeiss Microimaging Inc., Germany) and with appropriate configurations for multiple color acquisition. For quantitative and comparative imaging, identical image acquisition parameters were used.

**Immunohistostaining:** Metastatic melanoma (cutaneous, lymph nodes and lung localization) and nevi were obtained from patient biopsies from the Biobank of the University hospital of Nice, France. All patients provided informed written consent. Formalin-fixed tissues were embedded in paraffin. Sections (3 µm thick) were cut and subsequently immunostained using an automated immunostainer (Benchmark Ventana Medical System, Roche). All samples were stained for NF-kB2 or EZH2 using mouse monoclonal antibody against EZH2 (AC22; Cell Signalling #3147, 1:100) and Rabbit anti-NF-kB2 (18D10 Cell signaling #3017S, 1:300), followed by enzyme-conjugated secondary antibodies using the iview DAB kit detection. Stained sections were examined with Leica DM 2000 microscope (Leica microsystems, Wetzlar, Germany), images were acquired with Leica microsystems DFC295 camera (Leica microsystems, Wetzlar, Germany). The percentage of EZH2 or NF-kB2 positive cells and the staining intensity was evaluated for each tumor on the whole section, intensity was defined as negative (0), weak (+), moderate (++) or strong (+++).

**Chromatin immunoprecipitation:** ChIP assay was carried out to detect NF-kB2 binding to the EZH2 promoter locus, using magnachip kit (Millipore) according to manufacturer instruction using NF-kB2 (#4882 Cell Signaling) and IgG as negative control. As additional negative control, primers amplifying GAPDH promoter (Magna Millipore) were used. The enrichment of NF-kB2 immunoprecipitation on EZH2 promoter locus was calculated as the fold increase of the immunoprecipitated DNA compared with IgG-precipitated DNA. Results reported are representative of 3 independent immunoprecipitations.

**Statistical analysis:** Data are presented as averages ± SD of triplicate measurements of a representative experiment. Statistical significance of the experiments was evaluated using an unpaired two tailed student t-Test. [*] indicates a value of P < 0.05, [**] indicate a value of P<0.01.

Correlation analysis was performed with a Pearson correlation test, values above 0.3 were considered indicative of moderate correlation while values above 0.7 of strong correlation. Band quantification was obtained using ImageJ (NIH, Bethesda, MD).

**Sequence analysis of promoter:** The sequence analysis of EZH2 promoter was performed using TF-search (web site: www.cbrc.jp/research/db/TFSEARCH.html) for the NF-kB consensus motif. Threshold for sequence homology was set at 80%. The search returned two results located in the region from -100 to 0.

**Cristal violet staining:** Cells were washed with PBS, fixed in 3% PFA, washed then stained with Crystal violet 0.4%/ethanol 20% in PBS. Pictures were taken with Evos XL Core (Ozyme, France).

**Transcription Factor Decoys (TFD) preparation and treatment.** Double stranded TFDs were obtained by annealing at 95C two complementary single stranded oligos (purchased from Invitrogen). TFDs were designed by copying the NF-kB2 sequences discovered on the EZH2 promoter (i.e. p52 decoy referred as and TFD4 as previously described). For testing the ability of the TFDs to decrease EZH2 and induce senescence, 25000 cells from A375 melanoma cell line were plated in 6-well culture plates and transfected 24 hours later with 200nM TFDs using Lipofectamine LTX (Invitrogen) as transfecting agent, according to manufacturer protocol. If not otherwise described cells were incubated with the TFDs for 4 days prior harvesting for qPCR evaluation of EZH2 levels or Senescence associated-β-Gal assay. As non specific control, a scramble sequence was used.

### Results

**Pharmacologic NF-kB inhibition down-regulates EZH2:** To study the regulation of EZH2, we searched for hypothetical binding sites for transcription factors within EZH2 promoter. Two putative consensus motifs for NF-kB are localized in the region 0 to -100 close to the transcription starting site and E2F1 binding site (-400) (Figure 1A). We investigated if EZH2 expression was modulated by NF-kB inhibitors such as prostaglandin-J2 (PGJ2), Bay 11-7082 and sulfasalazine. First, we confirmed the inhibition of NF-kB activity in melanoma cells by those compounds (Figure 8A). Using quantitative real-time PCR (qRT-PCR), we observed in A375 melanoma cells a marked decrease of *EZH2* mRNA under treatment with these NF-kB inhibitors (Figure 1B). PGJ2 affected *EZH2* in a time and dose dependent manner starting within 6 hours of treatment (Figure 1C). We next confirmed the decrease of EZH2 at protein level by PGJ2 (Figure 1D), Bay 11-7082 and sulfasalazine (Figure 8B) using various metastatic melanoma cell lines carrying or not the B-RAF^{V600E} allele (Table 1). Taking into account that the melanoma cells show a strong genetic heterogeneity, it is of great interest to determine a molecular pathway that can be pharmacologically inhibited in all melanomas disregarding their mutational status. We cultured melanoma cells freshly extracted from nine melanoma patient's metastases and we characterized their mutational status (Table 1). Interestingly, we observed a consistent decrease of EZH2 in all those cells treated with PGJ2 (Figure 1E and 8C). Our findings indicate that the inhibition of NF-kB is sufficient to target EZH2 independently of the molecular signature of melanoma, and can thus likely be applied to a majority of melanoma patients.

**Table 1: Mutational status and activation of the MAPK pathway (related to p-ERK expression), for melanoma cell lines and cells isolated from patients used in the study.**

| **Cell lines** | **Localization** | **BRAF** | **NRAS** | **p53** | **CKIT** | **PTEN** | **p-ERK** |
|---|---|---|---|---|---|---|---|
| **A375** | N.A. | V600E | WT | WT | nd | WT | +++ |
| **501mel** | N.A. | V600E | WT | WT | nd | nd | + |
| **1205lu** | N.A. | V600E | WT | WT | WT | nd | +++ |
| **Mewo** | N.A. | WT | WT | mutant | nd | nd | ++ |
| **C10.02** | lymph | V600E | WT | nd | WT | nd | ++ |
| **C10.19** | subcut | V600E | WT | nd | WT | nd | +++ |
| **C09.01** | lymph | WT | WT | nd | WT | nd | +++ |
| **C10.08** | lymph | WT | WT | nd | WT | nd | + |
| **C12.06** | lymph | WT | WT | nd | WT | nd | ++ |
| **C10.21** | lymph | WT | WT | nd | WT | nd | ++ |
| **C10.05** | lymph | V600E | WT | nd | WT | nd | nd |
| **C10.07** | lymph | V600E | WT | nd | WT | nd | ++ |
| **C11.18** | lymph | nd | nd | nd | nd | nd | nd |

| | | | | | | | |
|---|---|---|---|---|---|---|---|
| Nd: non determined, N.A.: not applicable, Subcut: cutaneous metastasis, Lymph: lymph node metastasis | | | | | | | |

**NF-kB2 silencing down-regulates EZH2:** The NF-kB/REL family of transcription factors includes the subunits p50 (NF-KB1), p52 (NF-KB2), p65 (RELA), RELB, and c-REL. NF-kB proteins are normally sequestered in the cytoplasm by specific inhibitory proteins (IkBs). The NF-kB1 and NF-kB2 are translated as precursor proteins, p105 and p100, and also work as IkB-like molecule. Proteasome-mediated processing of p105 and p100 not only generates their respective mature and functional proteins, p50 and p52, but also results in the nuclear translocation of associated NF-kB members (Beinke and Ley, 2004; Hoffmann and Baltimore, 2006). We first investigated if the abolition of the "classical" (canonical) NF-kB pathway (RELA/p50) was responsible for the regulation of EZH2 transcription. Using RNA interference to silence RELA, we detected neither change in *EZH2* mRNA nor in EZH2 protein level (Figure 2A-2B).

We next investigated the role of the "non-canonical" NF-kB pathway in EZH2 regulation. This pathway is regulated by the control of NF-KB2 (p100) processing to the active p52 isoform which forms a heterodimer with RELB (Dejardin, 2006). Silencing NF-kB2 in melanoma cell lines triggered a significant and consistent down-regulation of *EZH2* mRNA and protein level in A375 cells (Figure 2C-2D). Similar results were obtained with Mel 501 melanoma cell line (Figure 9A-9B). Immunofluorescent staining performed in A375 cells showed a correlation between EZH2 and p100/p52 expression (Figure 2E). Interestingly, all tested patients' melanoma cells displayed a lower EZH2 level after NF-kB2 silencing independently of their mutational status at protein level (Figure 2F) and RNA level (Figure 9C). Consistently with those data, flow cytometry analysis of the level of H3k27me3, as readout of EZH2 activity, revealed a concomitant reduction (Figure 2G).

Therefore, these data show that the inhibition of the non-canonical NF-kB pathway significantly reduces EZH2 expression.

**NF-kB2 directly activates EZH2 promoter:** To further confirm the role of NF-kB2 in *EZH2* transcription, we analyzed the transcriptional activity of an EZH2 promoter reporter (EZH2-Renilla). When A375 melanoma cells were silenced for NF-kB2, a dramatic time-dependent reduction in promoter activity was observed, while RELA silencing did not trigger any modification (Figure 3A). Chromatin immunoprecipitation (ChIP) experiments using a p100/p52 antibody in A375 cells demonstrated a direct binding of p52 on EZH2 promoter (Figures 3B and 10A). Deletion of the core consensus motif of either one of the two hypothetical p52 binding sites by site directed mutagenesis dramatically abolished EZH2 promoter activity (Figure 3C).

**Importance of the non-canonical pathway to the overall NF-kB activity in melanoma compared with non-cancer cells:** Interestingly, using NF-kB luciferase reporter gene assays we observed a stronger decrease in total NF-kB activity when NF-kB2 was silenced compared with RELA silencing in A375 cells (Figure 3D). These results suggest a higher contribution of the non-canonical pathway than the classic one to the overall NF-kB activity in melanoma cells. We verified this hypothesis in five additional melanoma cells cultured from patients' biopsies. Again disregarding their mutational status, all tested cells displayed a stronger decrease in total NF-kB activity after NF-kB2 silencing compared with RELA silencing as opposed to non-cancer cells (Figure 10B). Both NF-kB2 and RELA were silenced with a similar efficiency in all the tested cells (Figures 10C-10D). Ratio of the effect of NF-kB2 versus RELA silencing on total NF-kB activity is depicted in Figure 3E.

**NF-kB2 overexpression increases EZH2 expression in melanoma and normal melanocytes:** Next, we showed that constitutive activation of the NF-kB2 pathway by forced expression of a CMV promoter-driven *NF-kB2* gene led to a significant increase in EZH2 promoter activity (Figure 3F) as well as in the protein level of EZH2 (Figure 3G). We then used the same procedure in normal human melanocytes (NHM) whose EZH2 level was barely detectable compared with melanoma cells (Figure 7A). NF-kB2 overexpression in melanocytes strongly increased EZH2 expression (Figure 3H). A significant shift in melanocyte proliferation was also seen compared with virus control-infected melanocytes with a 30% increase 3 days post-infection (Figure 10E). Those same cells were then seeded at a very low density; we observed a survival and growth advantage when NHM expressed NF-kB2 or EZH2 for 6 days (400% increase in cell number) (Figures 10F-G).

Overall, our findings reveal that the non-canonical NF-kB pathway plays a major contribution to the global NF-kB activity in melanoma, and plays a key role in regulating directly EZH2.

**NF-kB2 silencing down-regulates CDK2 and E2F1:** We undertook a detailed time course study in A375 cells transfected with NF-kB2 siRNA. As analyzed by qRT-PCR, *EZH2* mRNA content started decreasing within 16 hrs (Figure 4A) and protein level within 48 hours post NF-kB2 silencing (Figure 4B). We also investigated the expression level of E2F1, a known transcriptional regulator of EZH2, which is often deregulated in cancer. We observed a significant reduction in E2F1 for late time points in A375 (Figures 4A-4B and 11A-11B). In melanoma, as in many cancers, E2F1 activity is up regulated by CDK2-induced phosphorylation of the Retinoblastoma (Rb) protein triggering growth factor-independent cell replication (Halaban et al., 2000). Interestingly, NF-kB2 silencing led to a significant reduction in CDK2 mRNA and protein levels as well as a decreased phospho-Rb protein level (Figure 11B). Similar decrease of *E2F1* and *CDK2* were obtained with melanoma cells isolated from patients (Figure 11C). Inversely, NF-kB2 overexpression in melanocytes increased CDK2 and E2F1 (Figure S4D). We did not observe any decrease in CDK2 nor E2F1 level after silencing EZH2 in A375 cells, while CDK2 silencing significantly downregulated both E2F1 and EZH2 (Figures 4C-E). Furthermore we have not detected any binding site for NF-kB2 in CDK2 promoter. Those data suggest that NF-kB2 indirectly regulates both CDK2 and E2F1. As expected, given the reduced level of H3K27me3 observed post NF-kB2 silencing (Figure 2G), EZH2 down-regulation enhanced *p21* transcription (Figure 4C and E).

**NF-KB2 inhibition induces senescence:** We next investigated whether melanoma cells silenced for NF-kB2 were entering into senescence. Analysis of cell number revealed a significant decrease in cell number that was observable as early as 48 hours and reached 60% 4 days after NF-kB2 silencing in A375 cells (Figure 5A). Flow cytometry analysis showed a slight increase of the percentage of dead cells 4 days post-NF-kB2 silencing (Figure 12A) but a dramatic decrease in the proportion of cells in S phase and an increase in G2 (Figure 12B) concomitant with the raises in levels of *p21, p16* (high increase) and *p15*, *p27* (moderate increase) (Figure 12C) characteristic of a growth inhibition. Furthermore, cell size and granularity as determined by flow cytometry were significantly increased (Figure 5B-5C) as well as senescence associated β-Galactosidase (SA-β-Gal) activity (Figure 5D) and HP1γ expression (Figure 12D). Signs of senescence were also observed in all tested melanoma cells from patients (Figure 5D). A higher level of H3K9me3, a hallmark of senescence (Chandra et al., 2012) was detected by flow cytometry after EZH2 down-regulation (Figure 5E). We confirmed that CDK2 silencing also induced senescence (Figure S5E-S5H) (Giuliano et al., 2010).

**EZH2 overexpression prevents NF-kB2 silencing-induced senescence:** We next investigated the causal role of EZH2 down-regulation in the senescence induced by NF-kB2 silencing. We observed that forced expression of EZH2 in cells silenced for NF-kB2 prevented the increase of SA-β-Gal positive cells (Figure 5F) and the reduction of cell proliferation (Figure 12I). Finally, we investigated if NF-kB2 overexpression could prevent in normal melanocytes, the senescence induced by external stimuli such as H₂O₂ (Chen and Ames, 1994). Melanocytes infected with control virus prior to H2O2 treatment displayed a strong rate of SA-β-Gal positive cells while NF-kB2 forced expression significantly decreased H₂O₂-induced senescence (Figure 5G).

Those results indicate that NF-kB2 expression can prevent a stress-induced senescence in normal melanocytes and that the senescence program delayed by EZH2 in melanoma can be reactivated by silencing NF-kB2.

**NIK: a promising anti-melanoma target:** NF-KB2/P100 cleavage into p52 is dependent upon the presence of NF-kB inducing kinase (MAP3K14/NIK), a protein that is normally undetectable as it is co-translationally degraded by the TRAF3-TRAF2-cIAP E3 components(Sun). NIK overexpression has been reported in many cancers, including melanoma, and linked with poor prognosis (Thu and Richmond; Thu et al.). Similarly to NF-kB2 silencing, NIK silencing strongly decreased total NF-kB activity (Figure 13A-13C) and abolished EZH2 promoter transcriptional activity (Figure 13D). Using A375 and melanoma cells from patients, we confirmed EZH2 down-regulation at mRNA and protein level after NIK silencing (Figure 6A, 6C, 6D, Figure 13E). CDK2 and E2F1 were found decreased in almost all cell lines. Similarly to NF-kB2 and EZH2 silencing, NIK-silencing increased *p21* and *p16* levels (Figure 6B). As expected, NIK silencing induced a senescent phenotype, inhibiting melanoma cell growth in A375 and patients' cells (Figure 6E and Figure 13F) with minor cell death observed (Figure 13G), increased cell size and granularity (Figure 6F), and increased SA-β-Gal activity (Figure 6G). Similarly to NF-kB2 silencing, NIK-silencing-induced senescence was rescued by EZH2 overexpression (Figure 13H). Our results showed that NIK is responsible for NF-kB pathway activation and EZH2 overexpression in melanoma cells.

**NF-kB2 and EZH2 are correlated in cells and tissues extracted from patients' melanoma metastasis:** In total cell lysates of melanoma cell lines and patients' cells, we showed that increased level of NIK correlated with increased EZH2 expression (Figure 7A) independently of the mutational status and phospho-Erk expression. A Pearson correlation test for NIK/EZH2, p100/EZH2 and p52/EZH2 expression in 4 NHM, 3 melanoma cell lines and melanoma cells isolated from 5 of our patients, showed a significant correlation between the relative expression of these proteins (Figure 7B). We next undertook *in situ* immunohistostaining of NF-kB2 and EZH2 in melanoma tissues extracted from patients metastasis and found a strong correlated expression of both proteins while little or no expression for NF-kB2 nor EZH2 were observed in nevi (Figure 7C-7D and Table 2).

**Transcription factors decoys' mediated inhibition of the non-canonical NF-kB pathway activity reduce expression of EZH2 in cancer cells leading them undergoing senescence.** Among the possible mechanisms for inhibiting non-canonical NF-kB pathway activity, blocking the ability of NF-kB2 to bind on EZH2 promoter is surely appealing for its specificity and theoretical absence of unwanted side-effects. Transcription factors decoys are well known double stranded fragments of DNA that contains a binding site for a transcription factor. TFDs can thus reduce the transcriptional activity of the transcription factor by simply binding to it and preventing the activation of the transcription of its target gene. We designed several TFDs for NF-kB2 starting from the sequence of the binding site on EZH2 promoter. Among the one tested TFD1 and TFD4 were able to reduce of more than 50% the mRNA level of EZH2 when transfected inside A375 cells after 4days of treatment (Figure 14C). At the same time they were able to induce senescence in those cells thus replicating the action of a si-RNA directed against NF-kB2 (Figure 14A and 14B).

**Table 2. NF-kB2 and EZH2 expression in tissues from melanoma patients' metastasis versus nevi. Staining intensity and number of positive cells for NF-kB2 and EZH2 expression in melanoma samples analyzed in Figure 6. Localization of the metastatic sample and mutational status for BRAFV600E mutation are indicated.**

| **Samples** | **Diagnosis** | **NF-kB2** | | **EZH2** | | **BRAF** |
|---|---|---|---|---|---|---|
| | | **% CT** | **Intensity** | **% CT** | **Intensity** | |
| LH13-385 B | Nevus | 0% | | 0% | | N/A |
| LH13,1863 | Nevus | 0% | | 0% | | N/A |
| LH13-1948 B | Nevus | 0% | | 0% | | N/A |
| LH13,2004 P2 | Nevus | 0% | | 0% | | N/A |
| LH13,2005 P2 | Nevus | 0% | | 2% | + | N/A |
| LH12-1603 E | Cutaneous metastasis | 100% | ++ to +++ | 70% | + to +++ | Mutated V600E |
| LH12-3730 T | Cutaneous metastasis | 90% | ++ to +++ | 40% | + to ++ | Mutated V600E |
| LH12-3713 T | Cutaneous metastasis | 100% | + to ++ | 10% | + | WT |
| LH13-1890 C | Lymph node metastasis | 60% | + to ++ | 30% | + | Mutated V600E |
| LH13-1749 A | Lymph node metastasis | 60% | + to +++ | 50% | + to +++ | WT |
| LH13,1448 | Lymph node metastasis | 30% | + to ++ | 30% | + to ++ | WT |
| LH13,1310 | Lymph node metastasis | 70% | ++ to +++ | 70% | ++ to +++ | Mutated V600E |
| LH13-2041 1A3 | Lymph node metastasis | 60% | + to ++ | 50% | + to ++ | N/A |
| LH13-1281 | Lymph node metastasis | 90% | ++ to +++ | 70% | ++ to +++ | Mutated V600E |
| LH13-0369 | Lymph node metastasis | 90% | ++ to +++ | 50% | ++ | Mutated V600E |
| LH13-186 | Lymph node metastasis | 60% | + to +++ | 10% | + | WT |
| LH12-1922 G2 | Lymph node metastasis | 80% | + to ++ | 80% | + to +++ | Mutated V600E |
| LH12-1817 10 A3 | Lymph node metastasis | 90% | ++ to +++ | 30% | + to ++ | WT |
| LH12-1827 D | Lymph node metastasis | 100% | +++ | 80% | ++ to +++ | Mutated V600E |
| LH12-2643 1B | Lung metastasis | 90% | + to ++ | 30% | + to ++ | WT |
| LH12-1756 B3 | Lung metastasis | 100% | ++ to +++ | 70% | ++ to +++ | Mutated V600E |

| | | | | | | |
|---|---|---|---|---|---|---|
| %CT: Percentage of positive cells N/A: Non available WT: Wild type | | | | | | |

### DISCUSSION:

Using cell lines and cells cultured from melanoma patients' metastasis, we determined the critical role of the non-canonical NF-kB pathway (p100/p52 NF-kB2) in regulating EZH2 expression. Indeed, p52/p100 NF-kB subunit is aberrantly expressed in many tumor types. It has been implicated as a regulator of cell proliferation, and in normal cell transformation (Ciana et al., 1997). Here we have demonstrated that p52 binds directly to EZH2 promoter to activate its transcription. Deletion of p52 binding site on EZH2 promoter, or NF-kB2 silencing, significantly reduced EZH2 promoter activity and expression in melanoma cells. Thus, p52 acts as a key regulator of EZH2. NF-kB2 inhibition also down-regulates CDK2 and E2F1 that are main downstream effectors of cell cycle activating pathways and are often up-regulated in cancers including melanoma (Du et al., 2004). Furthermore we have shown the higher contribution of the non-canonical NF-kB signaling compared with the classical pathway to the overall NF-kB activity in melanoma cells. We have also emphasized the overexpression of p52, NIK and EZH2 in melanoma compared with melanocytes. Furthermore, we confirmed the *in vitro* correlated overexpression of NF-kB2 and EZH2 in tissue samples from patients' metastases. All together, these data underline a strong implication of the upregulation of the non-canonical NF-kB pathway for triggering EZH2 overexpression in melanoma. This concept can be likely applied to other cancers than melanoma given the increased non-canonical NF-kB activity reported in other solid cancers (Wharry et al., 2009).

Inhibition of the non-canonical pathway using NF-kB2 or NIK silencing induces senescence through the down-regulation of EZH2 which renders the transcription of p21 and p16 possible. Interestingly, we demonstrated that H2O2-mediated senescence in cultured melanocytes from healthy donor foreskin (NHM) is strongly reduced when melanocytes artificially overexpress NF-kB2, and consequently EZH2. In addition, NF-kB2 and EZH2 overexpression in NHM strongly increased their proliferation and may confer a survival advantage. Our findings suggest that an undesired sustained activity of the non-canonical NF-kB pathway in normal melanocytes would prevent a major defense mechanism against tumorigenesis such as senescence.

On a therapeutic point of view, inducing senescence in melanoma would be beneficial as it not only prevents cell cycle growth but also could trigger or potentiate the tumor immune response (Kang et al.; Xue et al., 2007). Among actual therapeutic strategies, enhancing the immune response against melanoma tumors seems to be the most promising so far. Ipilimumab, an anti-CTLA4 antibody increased the overall survival of stage 4 melanoma patients (Hodi et al.). Interestingly, long term responses can be achieved, but only 10% of the patients respond to treatment. Phase 2 studies with anti PD-L1 and anti PD-1 antibodies showed an increased response rate in17 and 28% of patients, respectively (Brahmer et al.; Topalian et al.). Still, a vast majority of patients will not respond to this approach. TNFα activates both the canonical and non-canonical NF-kB pathway (Razani et al.) and was recently demonstrated to be a crucial factor that causes reversible dedifferentiation and could participate to the resistance of melanoma against T-cell immunotherapy (Landsberg et al.). Thus inducing senescence through inhibition of the non-canonical pathway could substantially enhance the immune response and show beneficial effects in combination with anti-CTLA4 or anti-PD-1 or PD-L1. Finally, the senescence induced by NIK or NF-kB2 silencing was observable in all melanoma cells tested disregarding their mutation status or the activation of ERK. Thus, unlike BRAF or MEK targeted therapies that restrict the cohort of possible responding patients, drugs targeting the non-canonical pathway could be potentially used in all patients and applicable to a broad range of cancers.

These results shed light on the key role of non-canonical pathway of NF-kB in melanoma. The non-canonical pathway of NF-kB, and its upstream kinase NIK, thus, provide potential druggable targets for re-induction of senescence, that could be used alone, or to potentiate immune therapies, for melanoma but also for most cancers where the non-canonical NF-kB pathway is constitutively activated and EZH2 is overexpressed.

### EXAMPLE 2: IN VIVO EXPERIMENTS

### Material & Methods

**Production of cell line stably expressing an inducible shRNA against NFKB2:** TRIPZ doxycycline Inducible Lentiviral shRNA system (Dharmacon RHS4741) was used to produce stable A375 cells expressing an inducible shRNA construct against NFKB2. Lentiviral particles were produced in HEK293T cells following manufacturer instructions.

A375 cells were infected with a multiplicity of infection of 0.5 to ensure that only one lentivirus inserted into the cellular genome. 24hrs post infection cells that incorporated the viral genome were selected using 1µg/mL puromycin taking advantage of the gene of restistance present in the viral genome. Selection was continued for 6 days and, at the end, the expression of the shRNA was verified by adding to the cell medium 1µγ/mL Doxycicline everyday for 4 days. Western-blot and Q-PCR confirmed the decrease of proteins and mRNA levels for both NFKB2 and EZH2.

**siRNA transfection for animal experiments:** Approximately 2.5 X10^6 A375 cells were seeded and transfected after 24 hrs with 250µL of Lipefectamin RNAiMAx (lifetechnologies) and 250µL of 20µM solution of NFKB2 siRNA (mix of 4 different siRNAs with the sequences SEQ ID NO: 14, SEQ ID NO: 15, SEQ ID NO: 17; SEQ ID NO: 20) in one single solution) or control siRNA. 24 hrs post-transfection cells were harvested and resuspended in sterile PBS at a density of 2.5 X10^6 cells/mL. For each mouse a single injection of 100µL of cell suspension was performed (0.5 X10^6 cells/mouse).

**Animal experiments:** All animal experiments were performed according to the guidelines of the Institutional Animal Care and Use Committee and of the regional ethics committee (approval reference NCE/2013-73). 5 weeks old female athymic nude mice (Harlan) were injected subcutaneously with a single injection of 0.5 X10^6 A375 cells treated either with siRNA control (control group, 10 mice) or with siRNA for NFKB2 (treated group, 10 mice). For inducible SH in vivo experiments (3 groups control shRNA and NFKB2 shRNA #1 and #2, 7 mice per group) each mouse received 2 differently localized subcutaneous injections of 0.5 X10^6 cells, doxycycline was injected intraperitoneally everyday starting from the appearance of the tumor (4 days post injection) at a dose of 1.6mg/mouse. For all the mice, tumor size was measured daily with a caliper and growth progression was followed for 14 days. At the end of the period mice were sacrificed and the subcutaneous tumors were explanted, weighted and portions of them were included in tissuetek O.C.T. (sakura) or frozen in liquid nitrogen for Q-PCR or Western-blot analysis.

### Results

Both siRNA and inducible shRNA significantly reduced the volume and weight of melanoma tumors as compared to their respective control.

The analysis of RNA from the treated tumor showed a decrease of p52 and EZH2 expression as compared to tumor controls. As observed in culture cells, an increase of p21, p27 and more importantly of p16 was observed in the treated tumors.

### REFERENCES:

Throughout this application, various references describe the state of the art to which this invention pertains. The disclosures of these references are hereby incorporated by reference into the present disclosure.
Bang D, Wilson W, Ryan M, Yeh JJ, Baldwin AS. GSK-3α promotes oncogenic KRAS function in pancreatic cancer via TAK1-TAB stabilization and regulation of noncanonical NF-κB. Cancer Discov. 2013 Jun;3(6):690-703.
Bracken, A.P., Kleine-Kohlbrecher, D., Dietrich, N., Pasini, D., Gargiulo, G., Beekman, C., Theilgaard-Monch, K., Minucci, S., Porse, B.T., Marine, J.C., et al. (2007). The Polycomb group proteins bind throughout the INK4A-ARF locus and are disassociated in senescent cells. Genes & development 21, 525-530.
Bracken, A.P., Pasini, D., Capra, M., Prosperini, E., Colli, E., and Helin, K. (2003). EZH2 is downstream of the pRB-E2F pathway, essential for proliferation and amplified in cancer. The EMBO journal 22, 5323-5335.
Brahmer, J.R., Tykodi, S.S., Chow, L.Q., Hwu, W.J., Topalian, S.L., Hwu, P., Drake, C.G., Camacho, L.H., Kauh, J., Odunsi, K., et al. (2012). Safety and activity of anti-PD-Ll antibody in patients with advanced cancer. The New England journal of medicine 366, 2455-2465.
Chang, C.J., and Hung, M.C. (2012). The role of EZH2 in tumour progression. British journal of cancer 106, 243-247.
Ciana, P., Neri, A., Cappellini, C., Cavallo, F., Pomati, M., Chang, C.C., Maiolo, A.T., and Lombardi, L. (1997). Constitutive expression of lymphoma-associated NFKB-2/Lyt-10 proteins is tumorigenic in murine fibroblasts. Oncogene 14, 1805-1810.
Du, J., Widlund, H.R., Horstmann, M.A., Ramaswamy, S., Ross, K., Huber, W.E., Nishimura, E.K., Golub, T.R., and Fisher, D.E. (2004). Critical role of CDK2 for melanoma growth linked to its melanocyte-specific transcriptional regulation by MITF. Cancer cell 6, 565-576.
Fan, T., Jiang, S., Chung, N., Alikhan, A., Ni, C., Lee, C.C., and Hornyak, T.J. (2011). EZH2-dependent suppression of a cellular senescence phenotype in melanoma cells by inhibition of p21/CDKN1A expression. Mol Cancer Res 9, 418-429.
Hodi, F.S., O'Day, S.J., McDermott, D.F., Weber, R.W., Sosman, J.A., Haanen, J.B., Gonzalez, R., Robert, C., Schadendorf, D., Hassel, J.C., et al. (2010). Improved survival with ipilimumab in patients with metastatic melanoma. The New England journal of medicine 363, 711-723.
Hoenicke L, Zender L; Immune surveillance of senescent cells--biological significance in cancer- and non-cancer pathologies; Carcinogenesis. 2012 Jun;33(6):1123-6.
Kang, T.W., Yevsa, T., Woller, N., Hoenicke, L., Wuestefeld, T., Dauch, D., Hohmeyer, A., Gereke, M., Rudalska, R., Potapova, A., et al. (2011). Senescence surveillance of pre-malignant hepatocytes limits liver cancer development. Nature 479, 547-551.
Kheradmand Kia, S., Solaimani Kartalaei, P., Farahbakhshian, E., Pourfarzad, F., von Lindern, M., and Verrijzer, C.P. (2009). EZH2-dependent chromatin looping controls INK4a and INK4b, but not ARF, during human progenitor cell differentiation and cellular senescence. Epigenetics & chromatin 2, 16.
Larribere, L., Khaled, M., Tartare-Deckert, S., Busca, R., Luciano, F., Bille, K., Valony, G., Eychene, A., Auberger, P., Ortonne, J.P., et al. (2004). PI3K mediates protection against TRAIL-induced apoptosis in primary human melanocytes. Cell death and differentiation 11, 1084-1091.
Razani, B., Reichardt, A.D., and Cheng, G. (2011). Non-canonical NF-kappaB signaling activation and regulation: principles and perspectives. Immunological reviews 244, 44-54.
Topalian, S.L., Hodi, F.S., Brahmer, J.R., Gettinger, S.N., Smith, D.C., McDermott, D.F., Powderly, J.D., Carvajal, R.D., Sosman, J.A., Atkins, M.B., et al. (2012). Safety, activity, and immune correlates of anti-PD-1 antibody in cancer. The New England journal of medicine 366, 2443-2454.
Wharry, C.E., Haines, K.M., Carroll, R.G., and May, M.J. (2009). Constitutive non-canonical NFkappaB signaling in pancreatic cancer cells. Cancer Biol Ther 8, 1567-1576.
Xue, W., Zender, L., Miething, C., Dickins, R.A., Hernando, E., Krizhanovsky, V., Cordon-Cardo, C., and Lowe, S.W. (2007). Senescence and tumour clearance is triggered by p53 restoration in murine liver carcinomas. Nature 445, 656-660.

## Claims

1. An inhibitor of the non-canonical NF-κB pathway for use in a method for reducing or inhibiting EZH2 expression in cancer cells in a patient in need thereof, wherein said inhibitor is a NIK inhibitor or an inhibitor of NIK gene expression.

2. An inhibitor of the non-canonical NF-κB pathway for use in a method for inducing senescence in cancer cells in a patient in need thereof, wherein said inhibitor is a NIK inhibitor or an inhibitor of NIK gene expression.

3. An inhibitor of the non-canonical NF-κB pathway for use in a method for increasing tumor immunogenicity in a patient in need thereof, wherein said inhibitor is a NIK inhibitor or an inhibitor of NIK gene expression.

4. An inhibitor of the non-canonical NF-κB pathway for use in a method for increasing efficacy of a therapy with an immunomodulatory compound, wherein said inhibitor is a NIK inhibitor or an inhibitor of NIK gene expression.

5. An inhibitor of the non-canonical NF-κB pathway for use in a method for improving the survival time of a patient affected with a cancer and treated with an immunomodulatory compound, wherein said inhibitor is a NIK inhibitor or an inhibitor of NIK gene expression.

6. The inhibitor of the non-canonical NF-κB pathway for use according to any one claims 1 to 5, wherein the patient is a patient having melanoma.

7. The inhibitor of the non canonical NF-κB pathway for use according to any one of claims 1 to 6, wherein the NIK inhibitor is selected from the group consisting of 6-azaindole aminopyrimidine derivatives, alkynyl alcohol derivatives and pyrazoloisoquinoline derivatives.

8. A pharmaceutical composition or a kit-of-part comprising an inhibitor of the non-canonical NF-κB pathway and an immunomodulatory compound, wherein said inhibitor is a NIK inhibitor or an inhibitor of NIK gene expression.

9. The pharmaceutical composition or kit-of-part according to claim 8, wherein the immunomodulatory compound is selected from the group consisting of CTLA-4 antagonist, a PD-1 antagonist and a PD-L1 antagonist.

10. The pharmaceutical composition or kit-of-part according to claim 9, wherein the immunomodulatory compound is selected from the group consisting of an anti-CTLA-4 antibody, an anti-PD-Ll antibody and an anti-PD-1 antibody.


SEQUENCE LISTING

<110>  INSERM

<120>  COMPOSITIONS AND METHODS FOR INDUCING SENESCENCE IN CANCER CELLS

<130>  13121PASSERON

<160>  22

<170>  PatentIn version 3.3

<210>  1
<211>  1023
<212>  DNA
<213>  Homo sapiens

<400>  1
aaaaagtagt aacgggtccg gcggcagcgc gcgggccggg cgagcgtctc ccggcaaacg      60

cggcgccaca gctgagccga cctccggggc ccgcgccctc ccctccccgg gcaccactag     120

gagcggccag cccgggcctc ggctccgcgc gcggggaaac gagcgcggcg gttaaaaccg     180

ttaccacccc cgagttttga actggttcaa acttggcttc cagcacccgc cccgcccctc     240

ccccgcccgg gaactctgcg gcgccggttc ccgccaagag ccgccggcgc ttcgtcccgc     300

ccttcggccg gttcccgcca cctatcctcc ccgcctcccg tccgcggcgg gctccgggcc     360

cccgcgatgt ctcccggtcc ccgcgtgcct gcacaccgcc ttcctgagag gcgccgtgtg     420

ttcagcgaaa gaacaaagag acggcggcgg cgcttccaca cggccagtgg cgtcccttac     480

agcgaacccc gccgccgccc gcgcgcgcac gcgctgccag tgcccgcccg cccacgagcc     540

ctgagcgcac tctgcgtggg gctggctcgg cgcctccgag cccggcgggc cctgtgattg     600

gacgggcgcc cgcctcgcgt cccgccaatc ggggcggcgc ttgattgggc tgggggggcc     660

aaataaaagc gatggcgatt gggctgccgc gtttggcgct cggtccggtc gcgtccgaca     720

cccggtggga ctcagaaggc agtggagccc cggcggcggc ggcggcggcg cgcgggggcg     780

acgcgcggga acaacgcgag tcggcgcgcg ggacgaaggt aacgcgccgc tgcgggcggc     840

ccggccggcg gggctccggg agtgcgaacc gggcggcggc ggcggcgcca ggacctcccc     900

gccactgctg tgccggtccc gggtatcgcc gagcggggct caccggggcg ccgcgtttgt      960

aggcgtgcgg ggggtggagg gtgaggggag agcccccctc cccggaagga gctgtgagct    1020

tcg                                                                  1023

<210>  2
<211>  10
<212>  DNA
<213>  Artificial

<220>
<223>  EZH2-p52 (1) forward sequence

<400>  2
ggggctcacc                                                              10


<210>  3
<211>  10
<212>  DNA
<213>  Artificial

<220>
<223>  EZH2-p52 (1) reverse sequence

<400>  3
ccccgagtgg                                                              10


<210>  4
<211>  9
<212>  DNA
<213>  Artificial

<220>
<223>  EZH2-p52 (2) forward sequence

<400>  4
ggagagccc                                                                9


<210>  5
<211>  9
<212>  DNA
<213>  Artificial

<220>
<223>  EZH2-p52 (2) reverse sequence

<400>  5
cctctcggg                                                                9


<210>  6
<211>  11
<212>  DNA
<213>  Artificial

<220>
<223>  TFD1 EZH2-p52 (2) forward sequence

<400>  6
atcgggtctc c                                                            11


<210>  7
<211>  12
<212>  DNA
<213>  Artificial

<220>
<223>  TDF1 EZH2-p52 (2) reverse sequence

<400>  7
ggagagcccg at                                                           12

<210>  8
<211>  13
<212>  DNA
<213>  Artificial

<220>
<223>  TFD4 EZH2-p52 (1) forward sequence

<400>  8
atcggggctc acc                                                          13


<210>  9
<211>  13
<212>  DNA
<213>  Artificial

<220>
<223>  TDF4 EZH2-p52 (1) reverse sequence

<400>  9
ggtgagcccc gat                                                          13


<210>  10
<211>  9
<212>  PRT
<213>  Artificial

<220>
<223>  Peptide P1

<400>  10

Val Gln Arg Lys Arg Arg Lys Ala Leu
1               5


<210>  11
<211>  12
<212>  PRT
<213>  Artificial

<220>
<223>  Cell-penetrating peptide TAT peptide

<400>  11

Gly Arg Lys Lys Arg Arg Gln Arg Arg Arg Pro Gln
1               5                   10


<210>  12
<211>  25
<212>  RNA
<213>  Artificial

<220>
<223>  siRNA targeting NF-kB2

<400>  12
cccaggucug gaugguauua uugaa                                             25

<210>  13
<211>  25
<212>  RNA
<213>  Artificial

<220>
<223>  siRNA targeting NF-kB2

<400>  13
uucaauaaua ccauccagac cuggg                                          25


<210>  14
<211>  21
<212>  DNA
<213>  Artificial

<220>
<223>  siRNA targeting NF-kB2

<400>  14
aacccaggtc tggatggtat t                                              21


<210>  15
<211>  21
<212>  DNA
<213>  Artificial

<220>
<223>  siRNA targeting NF-kB2

<400>  15
ctggatggta ttattgaata t                                              21


<210>  16
<211>  21
<212>  DNA
<213>  Artificial

<220>
<223>  siRNA targeting NF-kB2

<400>  16
cggcgttgtc aacctcacca a                                              21


<210>  17
<211>  21
<212>  DNA
<213>  Artificial

<220>
<223>  siRNA targeting NF-kB2

<400>  17
aaggacatga ctgcccaatt t                                              21


<210>  18

<211>  25
<212>  RNA
<213>  Artificial

<220>
<223>  siRNA targeting NIK

<400>  18
gccaguccga gagucuugau cagau                                              25


<210>  19
<211>  25
<212>  RNA
<213>  Artificial

<220>
<223>  siRNA targeting NIK

<400>  19
aucugaucaa gacucucgga cuggc                                              25


<210>  20
<211>  21
<212>  DNA
<213>  Artificial

<220>
<223>  siRNA targeting NF-kB2

<400>  20
cacgggcaga ccagtgtcat t                                                  21


<210>  21
<211>  19
<212>  DNA
<213>  Artificial

<220>
<223>  shRNA targeting NF-kB2

<400>  21
ataagatttg aaataggtg                                                     19


<210>  22
<211>  19
<212>  DNA
<213>  Artificial

<220>
<223>  shRNA targeting NF-kB2

<400>  22
tcagttgcag aaacactgt                                                     19